(19) Europäisches Patentamt
European Patent Office
Office européen des brevets

(11) EP 3 494 127 B1

(12) FASCICULE DE BREVET EUROPEEN

(45) Date de publication et mention de la délivrance du brevet:
**28.09.2022 Bulletin 2022/39**

(21) Numéro de dépôt: **17758598.1**

(22) Date de dépôt: **04.08.2017**

(51) Classification Internationale des Brevets (IPC):
***C07K 14/005*** *(2006.01)* ***C12N 7/00*** *(2006.01)*
***C12N 15/63*** *(2006.01)* ***C12N 15/86*** *(2006.01)*

(52) Classification Coopérative des Brevets (CPC):
**C12N 7/00; C12N 15/86; C12N 15/902;**
C12N 2710/14143; C12N 2710/14151

(86) Numéro de dépôt international:
**PCT/FR2017/052191**

(87) Numéro de publication internationale:
**WO 2018/024998 (08.02.2018 Gazette 2018/06)**

(54) **SYSTÈME D'EXPRESSION BACULOVIRUS**

BACULOVIRUS EXPRESSIONSSYSTEM

BACULOVIRUS EXPRESSION SYSTEM

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **05.08.2016 FR 1657611**

(43) Date de publication de la demande:
**12.06.2019 Bulletin 2019/24**

(73) Titulaires:
- **Centre National de la Recherche Scientifique 75016 Paris (FR)**
- **Institut national de recherche pour l'agriculture, l'alimentation et l'environnement 75007 Paris (FR)**

(72) Inventeurs:
- **CERUTTI, Martine 30380 Saint Christol les Ales (FR)**
- **JULIANT, Sylvie 30380 Saint Christol les Ales (FR)**
- **THERY, Sylvie 30520 Saint Martin de Valgalgues (FR)**

(74) Mandataire: **Regimbeau 20, rue de Chazelles 75847 Paris Cedex 17 (FR)**

(56) Documents cités:
**WO-A2-01/12829 WO-A2-2010/055292**

- **POSSEE ROBERT D ET AL: "Generation of Baculovirus Vectors for the High-Throughput Production of Proteins in Insect Cells", BIOTECHNOLOGY AND BIOENGINEERING, WILEY & SONS, HOBOKEN, NJ, US, vol. 101, no. 6, 1 décembre 2008 (2008-12-01), pages 1115-1122, XP002552791, ISSN: 0006-3592, DOI: 10.1002/BIT.22002 [extrait le 2008-06-04]**
- **YUTA KANAI ET AL: "Multiple large foreign protein expression by a single recombinant baculovirus: A system for production of multivalent vaccines", PROTEIN EXPRESSION AND PURIFICATION., vol. 91, no. 1, 1 septembre 2013 (2013-09-01), pages 77-84, XP055324848, SAN DIEGO, CA. ISSN: 1046-5928, DOI: 10.1016/j.pep.2013.07.005**
- **POUL M-A ET AL: "Design of cassette baculovirus vectors for the production of therapeutic antibodies in insect cells", IMMUNOTECHNOLOGY, ELSEVIER SCIENCE PUBLISHERS BV, NL, vol. 1, no. 3, 1 décembre 1995 (1995-12-01), pages 189-196, XP004052721, ISSN: 1380-2933, DOI: 10.1016/1380-2933(95)00019-4**
- **LAKSHMI S VIJAYACHANDRAN ET AL: "Gene gymnastics: Synthetic biology for baculovirus expression vector system engineering", BIOENGINEERED, vol. 4, no. 5, 1 septembre 2013 (2013-09-01), pages 279-287, XP055145260, ISSN: 2165-5979, DOI: 10.4161/bioe.22966**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- **G. P. PIJLMAN: "Spontaneous excision of BAC vector sequences from bacmid-derived baculovirus expression vectors upon passage in insect cells", JOURNAL OF GENERAL VIROLOGY., vol. 84, no. 10, 1 octobre 2003 (2003-10-01), pages 2669-2678, XP055414049, GB ISSN: 0022-1317, DOI: 10.1099/vir.0.19438-0**

## Description

### Domaine technique

L'invention concerne un procédé de préparation d'un baculovirus recombinant comprenant au moins deux gènes exogènes, des jeux de recombinaison homologue mis en œuvre dans ce procédé de préparation, les baculovirus recombinants obtenus par ce procédé, ainsi que l'utilisation des baculovirus recombinants pour la production de polypeptides.

### Arrière-plan technologique

Les baculovirus sont une famille de virus en forme de bâtonnet, spécifiques des arthropodes, qui compte quatre genres (Alphabaculovirus, Betabaculovirus, Deltabaculovirus, Gammabaculovirus) regroupant 49 espèces. Les baculovirus ne sont pas capables de se répliquer dans les cellules de mammifères ou d'autres vertébrés.

Le génome des baculovirus est constitué d'une molécule d'ADN bicaténaire, circulaire, d'une taille comprise entre 80 et 180 kpb. Le génome des baculovirus s'associe à des protéines hautement basiques de 6,5 kDa, au sein d'une nucléocapside à symétrie hélicoïdale, qui contient une protéine de capside de 39 kDa. La taille du génome détermine la longueur de la nucléocapside. La nucléocapside est ensuite enfermée dans une enveloppe lipoprotéique pour former la particule virale ou virion. Ces structures peuvent être recouvertes d'une matrice cristalline ou polyédrique constituée essentiellement d'une seule protéine (la polyédrine) d'environ 30 kDa. Les polyèdres sont de grandes structures dont la taille varie de 1 à 15 $\mu$m de diamètre avec une enveloppe externe polysaccharidique qui confère une protection supplémentaire.

Les baculovirus, dont le génome a été modifié génétiquement, sont utilisés en biotechnologie pour la production de protéines recombinantes (i.e. baculovirus recombinants). Après pénétration dans une cellule d'insecte, ces baculovirus recombinants vont utiliser la machinerie de la cellule d'insecte pour produire la protéine recombinante.

Les baculovirus recombinants sont obtenus en insérant un ou plusieurs gènes provenant d'autres espèces (par exemple des êtres humains, d'autres vertébrés, plantes, bactéries et virus) dans le génome d'un baculovirus parent. Ces gènes sont placés sous le contrôle d'un promoteur viral ou cellulaire (par exemple le promoteur du gène de la polyédrine) pour générer un baculovirus recombinant. Le promoteur permet la transcription du gène étranger en ARN messager qui à son tour est traduit en protéine dans la cellule d'insecte infectée par le baculovirus recombinant. L'avantage d'utiliser ce système est que le niveau de production de la protéine recombinante dans les cellules d'insecte infectées par le baculovirus recombinant peut être très important. La protéine recombinante peut ensuite être purifiée à partir des cellules infectées si la protéine est intracellulaire ou bien à partir du milieu de culture si la protéine est sécrétée. Le système d'expression baculovirus est largement utilisé dans l'industrie et dans les laboratoires de recherche. En plus de l'importante productivité du système d'expression baculovirus, ce système est également très apprécié car il permet de produire des protéines recombinantes biologiquement actives. En effet, les cellules d'insectes permettent généralement d'obtenir des modifications post-traductionnelles appropriées.

Malgré tous ces avantages, le système d'expression baculovirus est difficile à mettre en œuvre à l'échelle industrielle pour la production de plusieurs séquences polypeptidiques recombinantes distinctes, par exemple des protéines comprenant plusieurs sous-unités distinctes, tels que les anticorps ou de couples de protéines virales. En effet, les procédés mis en œuvre pour la fabrication des baculovirus recombinants sont complexes et nécessitent un grand nombre d'étapes qui ne permettent pas d'obtenir des génomes de baculovirus recombinants homogènes et stables. L'homogénéité et la stabilité des génomes de baculovirus recombinants sont des critères nécessaires pour envisager un développement industriel.

Il a été décrit dans la demande internationale WO 01/12829 et dans l'article (Possee et al., 2008) un procédé de préparation d'un baculovirus recombinant, basé sur l'utilisation conjointe d'un baculovirus dans lequel un gène essentiel à la réplication virale est non-fonctionnel, et d'un vecteur de transfert comprenant une séquence nucléotidique permettant de restaurer la fonction d'un gène essentiel à la réplication, et un transgène codant pour un polypeptide d'intérêt. Ce procédé présente l'inconvénient de ne permettre d'intégrer qu'au plus deux transgènes d'intérêt, ces deux transgènes étant nécessairement intégrés dans un seul locus. Le procédé doit être répété plusieurs fois pour permettre l'intégration de transgènes supplémentaires.

Il existe donc un besoin de mettre au point des procédés faciles à mettre en œuvre et qui permettent de produire des protéines recombinantes comprenant plusieurs sous-unités distinctes, tels que les anticorps dans des systèmes d'expression baculovirus et qui peuvent notamment être développés à l'échelle industrielle.

Partant de ce constat, le demandeur a mis au point un procédé particulièrement efficace et facile à mettre en œuvre pour préparer des baculovirus recombinants homogènes et stables, et qui permet d'envisager un développement au niveau industriel, notamment pour la production de plusieurs séquences polypeptidiques recombinantes distinctes, notamment des protéines comprenant plusieurs sous-unités distinctes.

## Résumé de l'invention

Dans un premier aspect l'invention concerne un procédé pour préparer, dans une cellule d'insecte, un baculovirus recombinant comprenant n gènes exogènes, par multirecombinaison homologue entre :

a) un génome de baculovirus déficient pour la réplication dans lequel n gènes essentiels à la réplication virale sont non-fonctionnels ; et
b) n vecteurs de transfert comprenant chacun:

i) une séquence nucléotidique permettant de restaurer la fonction d'un des n gènes essentiels à la réplication virale non-fonctionnel,
ii) un des n gènes exogènes,

l'ensemble des séquences nucléotidiques i) des n vecteurs étant capables de restaurer la réplication du génome de baculovirus déficient pour la réplication ; n étant un nombre entier au moins égal à 2, et
le génome de baculovirus décifient pour la réplication et les n vecteurs de transfert étant introduits en une seule étape dans la cellule d'insecte.

Dans un deuxième aspect la présente description concerne un baculovirus recombinant comprenant (i.e. « baculovirus recombinant dont le génome comprend » ou « baculovirus recombinant dont le génome code ») n séquences nucléotidiques de formule (I) :
[gène exogène]-[séquence nucléotidique intercalaire]-[gène essentiel à la réplication virale fonctionnel] (I),
ladite séquence d'acides nucléiques intercalaire est constituée de 0 (zéro) à 600 pb, ledit gène essentiel à la réplication virale fonctionnel est sélectionné parmi 1629 (ORF9), Pk1 (ORF10), lef-1 (ORF14), ORF34, lef-11 (ORF37), p47 (ORF40), lef8 (ORF50), DNAJ domain (ORF51), ORF53, vp1054 (ORF54), Lef-9 (ORF62), DNA Pol (ORF65), lef-3 (ORF67), ORF73, ORF75, ORF81, p95 (ORF83), vp39 (ORF89), lef-4 (ORF90), p33 (ORF92), helicase (ORF95), vp80 (ORF104), ORF106-107, odv-ec43 (ORF109), gp64/67 (ORF128), ORF132, ORF133, odv-ec27 (ORF144), ORF146, ie1 (ORF147), lef-2 (ORF6); n étant un nombre entier au moins égal à 2.

Dans un troisième aspect la présente description concerne un jeu d'éléments de recombinaison homologue comprenant :

a) un génome de baculovirus déficient pour la réplication dans lequel n gènes essentiels à la réplication virale sont non-fonctionnels ;
b) n vecteurs de transfert comprenant chacun:

i) une séquence d'acides nucléiques permettant de restaurer la fonction d'un des n gènes essentiels à la réplication virale non-fonctionnel,
ii) éventuellement un gène exogène,

n étant un nombre entier au moins égal à 2.

Dans un quatrième aspect la présente description concerne une cellule comprenant un baculovirus recombinant de l'invention ou un jeu d'éléments de recombinaison homologue de l'invention.

Dans un cinquième aspect la présente description concerne l'utilisation d'un baculovirus recombinant de l'invention ou d'une cellule de l'invention pour la production de n polypeptides exogènes codés par n gènes exogènes.

## Description détaillée de l'invention

### Préparation du baculovirus recombinant

L'invention concerne un procédé pour préparer, dans une cellule d'insecte, un baculovirus recombinant comprenant n gènes exogènes, par multirecombinaison homologue entre :

a) un génome de baculovirus déficient pour la réplication dans lequel n gènes essentiels à la réplication virale sont non-fonctionnels ; et
b) n vecteurs de transfert comprenant chacun:

i) une séquence nucléotidique permettant de restaurer la fonction d'un des n gènes essentiels à la réplication virale non-fonctionnel,
ii) un des n gènes exogènes,

l'ensemble des séquences nucléotidiques i) des n vecteurs étant capables de restaurer la réplication du génome de baculovirus déficient pour la réplication ;

n étant un nombre entier au moins égal à 2, et
le génome de baculovirus décifient pour la réplication et les n vecteurs de transfert étant introduits en une seule étape dans la cellule d'insecte.

Dans le cadre de la présente invention, on entend designer par l'expression "génome de baculovirus" l'ensemble du matériel génétique d'un baculovirus, comprenant en particulier toutes les séquences codantes et ADN non codantes d'un baculovirus.

Dans le cadre de la présente invention, on entend designer par l'expression « génome de baculovirus déficient pour la réplication », un génome de baculovirus dans lequel n gènes essentiels à la réplication virale ont été soit délétés (entièrement ou partiellement) soit mutés de telle sorte que le génome de baculovirus a perdu sa capacité de réplication dans une cellule d'insecte. Par exemple, le gène essentiel à la réplication virale ne s'exprime plus ou il est transcrit puis traduit en une protéine non-fonctionnelle. Ainsi, les gènes délétés (entièrement ou partiellement) ou mutés sont appelés « gènes essentiels à la réplication virale non-fonctionnels ». Les génomes de baculovirus déficients pour la réplication virale sont fabriqués à partir de génomes de baculovirus parents en utilisant des techniques de biologie moléculaire bien connues de l'homme du métier, et permettant notamment l'insertion et/ou la délétion de séquences nucléotidiques dans le baculovirus parent. De préférence, le génome de baculovirus déficient pour la réplication virale comprend au moins une séquence nucléotidique qui lui permet de se répliquer au sein d'une cellule bactérienne. Ces séquences nucléotidiques ne sont pas des gènes exogènes au sens de l'invention. Un exemple d'élément de réplication bactérienne est la séquence nucléotidique « Mini-F ». De tels éléments de réplication sont bien connus dans l'état de la technique. La cellule bactérienne peut être *Escherichia coli.* Un génome de baculovirus qui comprend une séquence nucléotidique qui lui permet de se répliquer au sein d'une cellule bactérienne est connu sous la dénomination « BacMid ». De préférence, le génome de baculovirus déficient pour la réplication comprend également une ou plusieurs séquences nucléotidiques codant un ou plusieurs marqueurs de sélection permettant de sélectionner ou d'identifier les cellules bactériennes transfectées par le génome de baculovirus déficient pour la réplication. Ces séquences nucléotidiques ne sont pas des gènes exogènes au sens de l'invention. Un exemple de marqueurs de sélection est le gène de résistance à l'ampicilline, le gène de résistance à la kanamycine, le gène de résistance à l'hygromycine, le gène de résistance à la zéocine et/ou le gène de résistance à la tétracycline.

Dans un mode de réalisation particulier, le génome de baculovirus déficient pour la réplication est obtenu à partir d'un génome de baculovirus sélectionné parmi ou dérivé du génome de BmNPV, AcMNPV, ApNPV, BsSNPV, CfMNPV, EoSNPV, HaNPV, HzNPV, LdMNPV, MbMNPV, OpMNPV, SIMNPV, SeMNPV ou TeNPV, de préférence AcMNPV.

Dans un mode de réalisation préféré, le génome de baculovirus déficient pour la réplication est sous forme circulaire. Le procédé selon l'invention ne nécessite pas la linéarisation du génome déficient pour la réplication.

Dans le cadre de la présente invention, on entend designer par l'expression « génome de baculovirus recombinant », le génome de baculovirus issu de la recombinaison homologue entre le génome de baculovirus déficient pour la réplication virale et les n vecteurs de transfert.

Dans le cadre de la présente invention, on entend designer par l'expression « baculovirus recombinant », un baculovirus dont le génome est un génome de baculovirus recombinant, c'est-à-dire un baculovirus codant les n gènes exogènes. Le baculovirus recombinant est produit après réplication du génome de baculovirus recombinant dans la cellule d'insecte. Le baculovirus recombinant peut être sécrété et peut infecter une autre cellule d'insecte. De préférence, le baculovirus recombinant de l'invention est infectieux. Par « gène », on entend une séquence nucléotidique capable d'être transcrite en protéine ou en peptide d'intérêt.

Dans le cadre de la présente invention, on entend par « gène exogène » (ou « transgène »), un gène qui n'est naturellement pas présent dans le génome d'un baculovirus. Il peut s'agir par exemple de gènes humains, de gènes d'origine animale, de gènes viraux ou de gènes bactériens. De plus, un « gène exogène » au sens de l'invention n'est pas présent dans le génome de baculovirus déficient pour la réplication virale. Ainsi, les gènes qui ne sont naturellement pas présents dans le génome d'un baculovirus mais qui sont présents dans le génome de baculovirus déficient pour la réplication virale ne sont pas considérés comme des gènes exogènes au sens de la présente invention. En d'autres termes, les gènes exogènes sont portés par les vecteurs de transfert qui recombinent avec le génome de baculovirus déficient pour la réplication virale et qui se retrouvent ensuite intégrés dans le baculovirus recombinant.

Un gène exogène au sens de la présente invention est placé sous le contrôle d'éléments appropriés pour son expression dans la cellule d'insecte. Par "éléments appropriés", on entend l'ensemble des éléments nécessaires à sa transcription en ARN messager (ARNm) et à la traduction de l'ARNm en protéine. Parmi les éléments nécessaires à la transcription, le promoteur revêt une importance particulière. Il peut s'agir d'un promoteur constitutif ou d'un promoteur régulable et il peut être d'origine baculovirale ou d'origine arthropode (e.g. d'origine insecte). L'important est que le promoteur choisi soit adapté pour l'expression du gène exogène dans la cellule d'insecte. D'une façon générale, un promoteur en usage dans la présente invention, peut être modifié de manière à contenir des séquences régulatrices. A titre d'exemples de promoteurs, on peut citer le promoteur polyédrine, le promoteur P10, les promoteurs synthétiques dérivés des promoteurs polyédrine et P10, le promoteur IE1 du baculovirus CfMNPV, le promoteur IE1 du baculovirus LdMNPV, le promoteur gp64 du baculovirus OpMNPV, le promoteur IE1 du virus de crevette WSSV (White spot syndrome virus), le promoteur P9 du densovirus de *Junonia coenia* (JcDNV), le promoteur cellulaire A3 (actine 3) du vers à soie Bombyx mori. Dans un mode de réalisation particulier, un ou plusieurs des gènes exogènes est placé sous le contrôle d'un promoteur synthétique dérivé du promoteur P10 sauvage (SEQ ID NO :1), de préférence le promoteur synthétique P10S1A (SEQ ID NO : 2) ou P10S1B (SEQ ID NO :3).

Par « cassette d'expression », on entend une séquence nucléotidique généralement constituée d'un ou plusieurs gènes et des éléments appropriés pour son/leur expression, par exemple un gène exogène et les éléments appropriés pour son expression dans la cellule d'insecte.

Le baculovirus recombinant permet d'exprimer (i.e. de produire) les n gènes exogènes dans une cellule d'insecte. Dans un mode de réalisation particulier, la cellule d'insecte est choisie parmi Sf9, Sf21, Tn5-b14, les lignées cellulaires de lépidoptères sensibles au baculovirus AcMNPV ou les lignées Sf21, de préférence Sf9.

Les vecteurs de transfert peuvent également contenir une ou plusieurs séquences nucléotidiques qui leur permettent de se répliquer au sein d'une cellule bactérienne. Ils peuvent également contenir des gènes codant un marqueur de sélection permettant de sélectionner ou identifier les cellules bactériennes transformées avec un vecteur de transfert.

L'un des principaux avantages de la présente invention est que la capacité à se répliquer du génome de baculovirus déficient pour la réplication virale est restaurée par la recombinaison avec les n vecteurs de transfert. En effet, chacun des n vecteurs de transfert code, en plus d'un des n gènes exogènes, une séquence nucléotidique permettant de restaurer la fonction d'un des n gènes essentiels à la réplication virale non-fonctionnels. Ainsi, seule la recombinaison avec les n vecteurs de transfert est capable de restaurer la réplication du génome de baculovirus déficient pour la réplication. Ainsi le procédé de l'invention garantit que seuls les génomes de baculovirus recombinants contenant les n gènes exogènes pourront générer des baculovirus recombinants infectieux. Ce procédé évite d'avoir à utiliser des tests chronophages pour identifier les baculovirus recombinants contenant les n gènes exogènes.

Dans un mode de réalisation préféré, les gènes essentiels à la réplication virale sont choisis parmi 1629 (ORF9), Pk1 (ORF10), lef-1 (ORF14), ORF34, lef-11 (ORF37), p47 (ORF40), lef8 (ORF50), DNAJ domain (ORF51), ORF53, vp1054 (ORF54), Lef-9 (ORF62), DNA Pol (ORF65), lef-3 (ORF67), ORF73, ORF75, ORF81, p95 (ORF83), vp39 (ORF89), lef-4 (ORF90), p33 (ORF92), helicase (ORF95), vp80 (ORF104), ORF106-107, odv-ec43 (ORF109), gp64/67 (ORF128), ORF132, ORF133, odv-ec27 (ORF144), ORF146, ie1 (ORF147), lef-2 (ORF6). Ces gènes sont préférés car ils sont adjacents à un gène non-essentiel à la réplication virale. Ainsi, dans un mode de réalisation préféré, dans le génome de baculovirus déficient pour la réplication virale, les n gènes essentiels à la réplication virale non-fonctionnels sont chacun adjacents à un gène non-essentiel à la réplication virale. Comme détaillé tout au long de la présente demande, les n gènes exogènes recombinent chacun au niveau d'un gène non-essentiel à la réplication virale adjacent à un gène essentiel à la réplication virale non-fonctionnel. Etant donné que le gène non-essentiel à la réplication virale n'est pas essentiel pour la réplication du génome de baculovirus, cette recombinaison n'affecte pas la capacité du génome de baculovirus à se répliquer.

Au sens de la présente invention, l'expression « réplication » ou « réplication virale » s'entend par la réplication à la fois du génome de baculovirus et du baculovirus. Etant entendu que la réplication du génome de baculovirus dans une cellule d'insecte est essentielle à la réplication du baculovirus dans la cellule d'insecte. Ainsi, un gène essentiel à la réplication virale s'entend comme un gène essentiel à la réplication du baculovirus. La réplication du baculovirus dans la cellule d'insecte permet la génération de baculovirus infectieux. Ainsi, le procédé de l'invention permet de générer un baculovirus recombinant infectieux dans la cellule d'insecte.

Au sens de la présente invention, un gène essentiel à la réplication virale non-fonctionnel est adjacent à un gène non-essentiel à la réplication virale lorsque les deux gènes se succèdent ou se recouvrent partiellement sur le génome du baculovirus, de préférence aucun autre gène n'est compris entre le gène essentiel à la réplication virale non-fonctionnel et le gène non-essentiel à la réplication virale. Avantageusement, les deux gènes susmentionnés sont séparés par une séquence nucléotidique intercalaire, par exemple une séquence nucléotidique intercalaire non codante. En particulier, la séquence nucléotidique intercalaire a une longueur allant de 1 pb à 600 pb. Il est également possible qu'aucune séquence nucléotidique intercalaire (i.e. 0 pb) ne sépare les deux gènes susmentionnés, c'est-à-dire que les deux gènes susmentionnés sont accolés sur le génome du baculovirus ou se recouvrent partiellement. Alternativement, la séquence nucléotidique intercalaire peut comprendre un gène non-essentiel à la réplication virale.

Le demandeur s'est par ailleurs aperçu que le choix de gènes essentiels à la réplication virale non-fonctionnels adjacents à des gènes non-essentiels à la réplication virale était particulièrement avantageux et permettait d'obtenir des recombinaisons homologues homogènes et donc de préparer des baculovirus recombinants dont les génomes sont homogènes. Comme expliqué par ailleurs, seule une parfaite recombinaison des n vecteurs permet d'obtenir un baculovirus recombinant capable de se répliquer dans une cellule d'insecte. Aux fins de la présente invention, il est préférable que les génomes des baculovirus recombinants préparés selon l'invention soient homogènes à plus de 90%, avantageusement à plus de 95%, de préférence à plus de 99% et de manière tout à fait préférée aux environs de 100%, de préférence identique.

Dans un mode de réalisation particulier, le gène non-essentiel à la réplication virale est choisi parmi Ph (ORF 8), ORF11, ORF13, egt (ORF15), u-ubiquitin (ORF35), 39K (ORF36), ORF38, p43 (ORF39), lef-12 (ORF41), pcna (ORF49), ORF52, ORF55, Fp (ORF61), ORF63, gp37(ORF64), ORF68, ORF72, ORF74, ORF82, cg30 (ORF88), ORF91, pif4 (ORF96) he65 (ORF105), ORF108, ORF110, cathepsine (ORF127), p24 (ORF129), pp34 (ORF131), ORF134, ORF145, odv-e56 (ORF148), ORF5.

Avantageusement, le couple gène essentiel à la réplication virale/gène non-essentiel à la réplication virale est choisi parmi les couples listés dans le tableau suivant :

| Couple | Gène essentiel | Gène non essentiel | Espacement entre les 2 gènes |
|---|---|---|---|
| 1 | 1629 (ORF9) | Ph (ORF 8) | 29 bp |
| 2 | Pk1 (ORF10) | ORF11 | 163 bp |
| 3 | lef-1 (ORF14) | ORF13<br>ou egt (ORF15) | Chevauchement<br>112 bp |
| 4 | ORF34 | v-ubiquitin (ORF35) | 20 bp |
| 5 | lef-11 (ORF37) | 39K (ORF36)<br>ou ORF38 | Chevauchement<br>Chevauchement |
| 6 | p47 (ORF40) | p43 (ORF39)<br>ou lef-12 (ORF41) | 7 bp<br>Chevauchement |
| 7 | Lef8 (ORF50) | pcna (ORF49) | 109 bp |
| 8 | DNAJ domain (ORF51) | ORF52 | 202 bp |
| 9 | ORF53 | ORF52 | 1 bp |
| 10 | vp1054 (ORF54) | ORF55 | 91 bp |
| 11 | Lef-9 (ORF62) | Fp (ORF61)<br>ou ORF63 | 26 bp<br>60 bp |
| 12 | DNA Pol (ORF65) | gp37(ORF64) | 137 bp |
| 13 | lef-3 (ORF67) | ORF68 | Chevauchement |
| 14 | ORF73 | ORF72 | 8 bp |
| 15 | ORF75 | ORF74 | 17 bp |
| 16 | ORF81 | ORF82 | Chevauchement |
| 17 | p95 (ORF83) | ORF82 | Chevauchement |
| 18 | vp39 (ORF89) | cg30 (ORF88) | 2 bp |
| 19 | lef-4 (ORF90) | ORF91 | 0 bp |
| 20 | p33 (ORF92) | ORF91 | 37 bp |
| 21 | helicase (ORF95) | pif-4 (ORF96) | Chevauchement |
| 22 | vp80 (ORF104) | he65 (ORF105) | 27 bp |

| | | | |
|---|---|---|---|
| 23 | ORF106-107 | he65 (ORF105) | 544 bp |
| 24 | odv-ec43 (ORF109) | ORF108 ou ORF110 | 11 bp 35 bp |
| 25 | gp64/67 (ORF128) | cathepsine (ORF127) ou p24 (ORF129) | 224 bp 182 bp |
| 26 | ORF132 | pp34 (ORF131) | 211 bp |
| 27 | ORF133 | ORF134 | 50 bp |
| 28 | odv-ec27 (ORF144) | ORF145 | 69 bp |
| 29 | ORF146 | ORF145 | Chevauchement |
| 30 | ie1 (ORF147) | odv-e56 (ORF148) | 61 bp |
| 31 | lef-2 (ORF6) | ORF5 | Chevauchement |

Dans le cadre de la présente invention, on entend designer par l'expression « recombinaison homologue », l'échange d'information génétique entre deux séquences nucléotidiques différentes, nécessitant la présence de séquences homologues entre les deux séquences nucléotidiques différentes. Dans le cadre de la présente invention, la recombinaison se fait dans la cellule d'insecte entre (a) un génome de baculovirus déficient pour la réplication dans lequel n gènes essentiels à la réplication virale sont non-fonctionnels et (b) les n vecteurs de transfert. La recombinaison dans le cadre de la présente invention se fait en une seule étape dans la cellule d'insecte. C'est-à-dire que la recombinaison des n gènes exogènes avec le génome de baculovirus déficient pour la réplication se fait de manière simultanée ou quasi-simultanée dans la cellule d'insecte.

Le procédé selon l'invention met en œuvre un mécanisme de recombinaison homologue intermoléculaire. D'une manière générale, le mécanisme de recombinaison homologue consiste en l'échange de séquences nucléotidiques homologues entre le génome de baculovirus déficient pour la réplication et les n vecteurs de transfert. Ces séquences nucléotidiques peuvent être identiques ou substantiellement homologues. Dans un mode de réalisation particulièrement avantageux, les vecteurs de transfert comprennent, de part et d'autre de la cassette d'expression du gène exogène, des séquences flanquantes homologues au génome de baculovirus déficient pour la réplication. Le degré d'homologie des séquences flanquantes avec la partie correspondante du génome de baculovirus déficient pour la réplication peut être variable mais doit être suffisant pour permettre la recombinaison intermoléculaire. Aux fins de la présente invention, il est préférable qu'il soit supérieur à 70%, avantageusement supérieur à 80%, de préférence supérieur à 90% et de manière tout à fait préférée aux environs de 100%, de préférence identique. De plus, une courte région d'homologie peut être suffisante, c'est-à-dire au moins 10 nucléotides (ou paires de bases) consécutifs communs entre les séquences flanquantes et leurs séquences homologues dans le génome de baculovirus déficient pour la réplication. Dans le cadre de la présente invention, la longueur des séquences flanquantes peut aller de 10 pb (i.e. 10 paires de bases) à 10 kb (i.e. 10 000 paires de bases), avantageusement allant de 100 pb à 6 kb, de préférence allant de 200 pb à 6 kb et, de manière tout à fait préférée allant de 400 pb à 6 kb. Ainsi, le matériel génétique localisé entre les séquences flanquantes des n vecteurs de transfert remplace le matériel génétique localisé entre les deux séquences homologues aux séquences flanquantes du génome de baculovirus déficient pour la réplication. Cet échange intermoléculaire permet de générer un baculovirus recombinant infectieux dans la cellule d'insecte. Selon l'invention, l'ensemble des séquences nucléotidiques i) (i.e. les séquences nucléotidiques permettant de restaurer la fonction des n gènes essentiels à la réplication virale non-fonctionnels) des n vecteurs de transfert sont capables de restaurer la réplication du génome de baculovirus déficient pour la réplication. En effet, l'échange intermoléculaire permet de restaurer la fonction des n gènes essentiels à la réplication virale non-fonctionnels. En d'autres termes, la restauration de la fonction des n gènes essentiels à la réplication virale non-fonctionnels se fait lorsque la recombinaison homologue se produit correctement. Cela vient du fait que les n vecteurs de transfert comprennent chacun une séquence nucléotidique permettant de restaurer la fonction d'un des n gènes essentiels à la réplication virale non-fonctionnels.

Par exemple, lorsque n=2, un génome de baculovirus déficient pour la réplication dans lequel deux gènes essentiels à la réplication virale sont non-fonctionnels recombine avec deux vecteurs de transfert qui comprennent chacun une séquence nucléotidique permettant de restaurer la fonction d'un des deux gènes essentiels à la réplication virale non-fonctionnel. Ce qui veut dire que la recombinaison avec le premier vecteur de transfert permet de restaurer la fonction d'un premier gène essentiel à la réplication virale non-fonctionnel et la recombinaison avec le deuxième vecteur de transfert permet de restaurer la fonction d'un deuxième gène essentiel à la réplication virale non-fonctionnel. Ainsi, seule la recombinaison des deux vecteurs de transfert avec le génome de baculovirus déficient pour la réplication permet de restaurer la fonction des deux gènes essentiels à la réplication virale non-fonctionnels et donc de restaurer la réplication du génome de baculovirus déficient pour la réplication. Cette restauration permet de générer des baculovirus recombinants infectieux dans la cellule d'insecte.

Ainsi, selon le procédé de l'invention, la recombinaison avec les n vecteurs de transfert, ou multirecombinaison, est nécessaire pour restaurer la réplication du génome de baculovirus déficient pour la réplication.

De manière surprenante, les inventeurs ont mis en évidence que la multirecombinaison pouvait se faire en une seule étape dans la cellule d'insecte. Ceci est particulièrement avantageux puisque le génome de baculovirus déficient pour la réplication et les n vecteurs de transfert peuvent être introduits en même temps dans la cellule d'insecte, c'est-à-dire que le génome de baculovirus déficient pour la réplication et les vecteurs de transfert sont introduits simultanément dans la cellule d'insecte, en d'autres termes le génome de baculovirus déficient pour la réplication et les vecteurs de transfert sont introduits en une seule étape dans la cellule d'insecte. La multirecombinaison en une seule étape lors du procédé selon l'invention est donc très facile à mettre en œuvre et permet d'obtenir des génomes de baculovirus recombinants homogènes, c'est-à-dire que l'homologie de la séquence nucléotidique de chaque génome de baculovirus recombinant produit est supérieure à 90%, de préférence supérieure à 95%, de préférence supérieure à 99%, de préférence aux environs de 100%, de préférence égale à 100%.

Le procédé selon l'invention permet de préparer un baculovirus recombinant exprimant (i.e. produisant) n polypeptides exogènes. Les n polypeptides exogènes forment une seule protéine ou plusieurs protéines. On entend par « polypeptide » une chaine d'acides aminés reliés par des liaisons peptidiques. Par exemple, un polypeptide peut être une protéine, une sous-unité de protéine, un fragment de protéine ou simplement une chaine d'acides aminés. Généralement, un polypeptide est formé d'au moins 10 acides aminés.

Dans un mode de réalisation particulier, les n polypeptides exogènes forment plusieurs protéines. Il peut s'agir de protéines comprenant une seule chaine polypeptidique, plusieurs sous-unités identiques ou plusieurs sous-unités distinctes. Le nombre de protéines produites peut être égal ou inférieur à n. Par exemple, trois polypeptides exogènes (n=3) peuvent former (i) une protéine comprenant une seule chaine polypeptidique et une protéine comprenant deux sous-unités distinctes, (ii) trois protéines comprenant une seule chaine polypeptidique, (iii) une protéine comprenant plusieurs sous-unités identiques et une protéine comprenant deux sous-unités distinctes ou (iv) trois protéines comprenant plusieurs sous-unités identiques.

Dans un autre mode de réalisation particulier, les n polypeptides exogènes forment une seule protéine. Dans ce mode de réalisation, la protéine comprend généralement n sous-unités distinctes.

Le procédé selon l'invention est particulièrement avantageux pour préparer un baculovirus recombinant exprimant (i.e. produisant) une protéine exogène comprenant plusieurs sous-unités distinctes, par exemple une protéine n'ayant sa fonction que lorsqu'elle comprend toutes ses sous-unités. Les sous-unités sont généralement liées entre elles par des liaisons non-covalentes (e.g. liaisons hydrophobes) et/ou des liaisons covalentes (e.g. ponts disulfures entre deux cystéines).

Dans un mode de réalisation particulier, le procédé selon l'invention permet de préparer facilement et rapidement un baculovirus recombinant comprenant n gènes exogènes distincts qui codent une seule protéine comprenant n sous-unités distinctes. On entend par « gènes exogènes distincts », des gènes n'ayant pas la même séquence nucléotidique. On entend par « sous-unités distinctes », des séquences peptidiques n'ayant pas la même séquence en acides aminés. Ainsi, une « protéine comprenant n sous-unités distinctes », comprend n sous-unités distinctes liées entre elles par des liaisons non-covalentes et/ou des liaisons covalentes. Dans ce mode de réalisation, le nombre de vecteurs de transfert dépendra du nombre de sous-unités distinctes que comprend la protéine. Par exemple, deux vecteurs de transfert seront utilisés pour une protéine comprenant deux sous-unités distinctes, trois vecteurs de transfert seront utilisés pour une protéine comprenant trois sous-unités distinctes, etc. Ainsi, chaque vecteur de transfert comprend un gène exogène différent des gènes exogènes compris dans les autres vecteurs de transfert. Le baculovirus recombinant de l'invention ne peut comprendre l'ensemble de la protéine d'intérêt (c'est-à-dire l'ensemble des sous-unités distinctes qui constituent la protéine d'intérêt) uniquement si la recombinaison se fait entre le génome de baculovirus déficient pour la réplication et les n vecteurs de transfert.

Selon l'invention, n est un nombre entier au moins égal à 2, par exemple un nombre entier allant de 2 à 30, par exemple allant de 2 à 10. Par exemple, pour une protéine comprenant plusieurs sous-unités distinctes, la valeur de n correspondra au nombre de sous-unités distinctes qui constituent ladite protéine.

Dans un mode de réalisation particulier, n=2 et le baculovirus recombinant code 2 gènes exogènes qui codent une protéine comprenant deux sous-unités, avantageusement 2 gènes exogènes distincts qui codent une protéine comprenant 2 sous-unités distinctes. De préférence, la protéine est un anticorps, un complexe protéique formant des Virus-Like-Particles (VLP), un couple de protéines virales, une hormone peptidique, de préférence un anticorps.

Dans un mode de réalisation particulier, n=3 et le baculovirus recombinant comprend 3 gènes exogènes qui codent une protéine comprenant trois sous-unités, avantageusement 3 gènes exogènes distincts qui codent une protéine comprenant 3 sous-unités distinctes. De préférence, la protéine est un anticorps bispécifique, un ensemble de trois protéines virales, un complexe multienzymatique, un complexe protéique, un complexe protéique formant des VLP, de préférence un anticorps bispécifique.

Le terme « anticorps » est utilisé ici dans le sens le plus large et englobe diverses structures d'anticorps largement décrites dans la littérature, y compris, mais sans s'y limiter, les anticorps quelle que soit leur origine, les anticorps monoclonaux, les anticorps polyclonaux et les fragments d'anticorps tant qu'ils présentent l'activité souhaitée (par exemple la liaison à l'antigène). L'anticorps est une IgA, IgD, IgE, IgG ou une IgM. Des exemples de fragments d'anticorps comprennent, mais sans s'y limiter, des fragments Fv, Fab, Fab', Fab'-SH, F(ab')2, Fc ; les diabodies; scFv/Fc ; les anticorps de type camélidés (par exemple les VHH); des molécules d'anticorps à chaîne unique (par exemple scFv).

Avantageusement, le baculovirus recombinant préparé selon le procédé de l'invention ne comprend pas de séquence d'acides nucléiques qui lui permet de se répliquer au sein d'une cellule bactérienne. En particulier, la séquence d'acides nucléiques qui permet de répliquer le génome de baculovirus déficient pour la réplication au sein d'une cellule bactérienne est éliminée lors de la recombinaison homologue dans la cellule d'insecte.

L'introduction dans une cellule d'insecte des vecteurs de transfert et du génome de baculovirus déficient pour la réplication est réalisée selon les techniques générales largement décrites dans l'art antérieur. On peut citer notamment la technique au phosphate de calcium, la technique au DEAE dextran, l'électroporation, les méthodes basées sur les chocs osmotiques, la microinjection ou les méthodes basées sur l'emploi de liposomes, de préférence la lipofection. Dans le cadre de la présente invention, les vecteurs de transfert et le génome de baculovirus déficient pour la réplication sont introduits en même temps dans la cellule d'insecte, c'est-à-dire que les vecteurs de transfert et le génome de baculovirus déficient pour la réplication sont introduits simultanément dans la cellule d'insecte, en d'autres termes les vecteurs de transfert et le génome de baculovirus déficient pour la réplication sont introduits en une seule étape.

Dans le cadre d'un procédé selon l'invention, les quantités de génome de baculovirus déficient pour la réplication et de vecteurs de transfert introduits dans la cellule d'insecte peuvent varier. On préfère employer une quantité 5 fois plus importante de chacun des vecteurs de transfert par rapport à la quantité de génome de baculovirus déficient pour la réplication.

Un autre avantage de la présente invention est que le génome de baculovirus déficient pour la réplication est introduit sous forme circulaire, c'est-à-dire sans étape de linéarisation au préalable. Cette étape de linéarisation est inutile puisque le génome de baculovirus est déficient pour la réplication, même sous forme circulaire, puisqu'il comprend des gènes essentiels à la réplication virale non-fonctionnels. L'absence d'étape de linéarisation est un autre avantage majeur de la présente invention.

Baculovirus recombinant

La présente description concerne également un baculovirus recombinant comprenant (i.e. « baculovirus recombinant dont le génome comprend » ou « baculovirus recombinant dont le génome code ») n séquences nucléotidiques de formule (I) :

[gène exogène]-[séquence nucléotidique intercalaire]-[gène essentiel à la réplication virale fonctionnel] (I),

ladite séquence d'acides nucléiques intercalaire est constituée de 0 (zéro) à 600 pb, ledit gène essentiel à la réplication virale fonctionnel est sélectionné parmi 1629 (ORF9), Pk1 (ORF10), lef-1 (ORF14), ORF34, lef-11 (ORF37), p47 (ORF40), lef8 (ORF50), DNAJ domain (ORF51), ORF53, vp1054 (ORF54), Lef-9 (ORF62), DNA Pol (ORF65), lef-3 (ORF67), ORF73, ORF75, ORF81, p95 (ORF83), vp39 (ORF89), lef-4 (ORF90), p33 (ORF92), helicase (ORF95), vp80 (ORF104), ORF106-107, odv-ec43 (ORF109), gp64/67 (ORF128), ORF132, ORF133, odv-ec27 (ORF144), ORF146, ie1 (ORF147), lef-2 (ORF6); n étant un nombre entier au moins égal à 2.

Avantageusement, le baculovirus recombinant ne comprend pas de séquence d'acides nucléiques qui lui permet de se répliquer au sein d'une cellule bactérienne. Il a en effet été démontré que l'absence d'une telle séquence permettait d'augmenter la stabilité du baculovirus recombinant, comparé à un baculovirus recombinant comportant une telle séquence (Pijlman et al (2003), Spontaneous excision of BAC vector sequences from bacmid-derived baculovirus expression vectors upon passage in insect cells. Journal of General Virology).

Avantageusement, le baculovirus recombinant ne comprend pas n gènes non-essentiels à la réplication virale choisis parmi Ph (ORF 8), ORF11, ORF13, egt (ORF15), v-ubiquitin (ORF35), 39K (ORF36), ORF38, p43 (ORF39), lef-12 (ORF41), pcna (ORF49), ORF52, ORF55, Fp (ORF61), ORF63, gp37(ORF64), ORF68, ORF72, ORF74, ORF82, cg30 (ORF88), ORF91, pif-4 (ORF96) he65 (ORF105), ORF108, ORF110, Cathepsine (ORF127), p24 (ORF129), pp34 (ORF131), ORF134, ORF145, odv-e56 (ORF148), ORF5. Avantageusement, un des n gènes non-essentiels à la réplication virale est le gène codant la cathepsine car il a été montré que la cathepsine peut avoir un effet délétère sur les polypeptides exogènes produits.

n est un nombre entier au moins égal à 2, par exemple un nombre entier allant de 2 à 30, par exemple allant de 2 à 10, comme détaillé dans la partie « préparation du baculovirus recombinant » ci-dessus.

La présente description concerne également un baculovirus recombinant, susceptible d'être obtenu selon le procédé de préparation de l'invention, comprenant n séquences nucléotidiques de formule (I) :

[gène exogène]-[séquence nucléotidique intercalaire]-[gène essentiel à la réplication virale fonctionnel] (I),

ladite séquence d'acides nucléiques intercalaire est constituée de 0 (zéro) à 600 pb, ledit gène essentiel à la réplication virale fonctionnel est sélectionné parmi 1629 (ORF9), Pk1 (ORF10), lef-1 (ORF14), ORF34, lef-11 (ORF37), p47 (ORF40), lef8 (ORF50), DNAJ domain (ORF51), ORF53, vp1054 (ORF54), Lef-9 (ORF62), DNA Pol (ORF65), lef-3 (ORF67), ORF73, ORF75, ORF81, p95 (ORF83), vp39 (ORF89), lef-4 (ORF90), p33 (ORF92), helicase (ORF95), vp80 (ORF104), ORF106-107, odv-ec43 (ORF109), gp64/67 (ORF128), ORF132, ORF133, odv-ec27 (ORF144), ORF146, ie1 (ORF147), lef-2 (ORF6); n étant un nombre entier au moins égal à 2.

Avantageusement, les n séquences nucléotidiques sont réparties sur tout le génome du baculovirus recombinant, ce qui permet d'en améliorer la stabilité. Les n gènes exogènes choisis sont donc suffisamment espacés sur le génome du baculovirus. Cette caractéristique est implicite puisque la répartition sur le génome est liée aux couples « gène essentiel/gène non essentiel ».

Avantageusement, et cela est inhérent au procédé de préparation selon l'invention, les n séquences nucléotidiques de formule (I) ne sont pas dupliquées sur le génome du baculovirus recombinant. Il a en effet été démontré une diminution de la stabilité du baculovirus recombinant lorsque les séquences sont dupliquées sur le génome (données non-présentées).

<u>Jeu d'éléments de recombinaison homologue</u>

La présente description concerne également un jeu d'éléments de recombinaison homologue comprenant :

a) un génome de baculovirus déficient pour la réplication dans lequel n gènes essentiels à la réplication virale sont non-fonctionnels ;
b) n vecteurs de transfert comprenant chacun:

   i) une séquence d'acides nucléiques permettant de restaurer la fonction d'un des n gènes essentiels à la réplication virale non-fonctionnel,
   ii) éventuellement un gène exogène,

n étant un nombre entier au moins égal à 2.

Avantageusement, les gènes essentiels à la réplication virale non-fonctionnels sont chacun adjacents à un gène non-essentiel à la réplication virale, comme décrit dans la partie « préparation du baculovirus recombinant » ci-dessus.

Dans un mode de réalisation particulier, les vecteurs de transfert comprennent, de part et d'autre de la cassette d'expression du gène exogène, des séquences flanquantes homologues au génome de baculovirus déficient pour la réplication. Avantageusement, les séquences flanquantes de chacun des vecteurs de transfert sont homologues à tout ou partie dudit gène essentiel à la réplication virale non-fonctionnel et à tout ou partie dudit gène non-essentiel à la réplication virale. Avantageusement, les séquences flanquantes ont une longueur qui peut aller de 10 pb (i.e. 10 paires de bases) à 10 kb (i.e. 10 000 paires de bases), avantageusement allant de 100 pb à 6 kb, de préférence allant de 200 pb à 6 kb et, de manière tout à fait préférée allant de 400 pb à 6 kb. Les séquences flanquantes sont décrites en détail dans la partie « préparation du baculovirus recombinant » ci-dessus.

Dans un mode de réalisation particulier, les n vecteurs de transfert comprennent chacun un gène exogène.

Dans un mode de réalisation particulier, les n gènes exogènes codent n polypeptides exogènes. Les n polypeptides exogènes forment une seule protéine ou plusieurs protéines, comme détaillé dans la partie « préparation du baculovirus recombinant » ci-dessus.

Dans un mode de réalisation particulier, n=2 et les 2 gènes exogènes codent une protéine comprenant deux sous-unités, avantageusement 2 gènes exogènes distincts qui codent une protéine comprenant de 2 sous-unités distinctes. De préférence, la protéine est un anticorps monospécifique, une VLP, un couple de protéines virales, une hormone peptidique, de préférence un anticorps.

Dans un mode de réalisation particulier, n=3 et les 3 gènes exogènes codent une protéine comprenant trois sous-unités, avantageusement 3 gènes exogènes distincts qui codent une protéine comprenant de 3 sous-unités distinctes. De préférence, la protéine est un anticorps bispécifique, un ensemble de trois protéines virales, un complexe multienzymatique, un complexe protéique, une VLP composée de 3 protéines distinctes, de préférence un anticorps bispécifique.

Les « gènes essentiels à la réplication virale » sont décrits dans la partie « préparation du baculovirus recombinant » ci-dessus.

Dans un mode de réalisation particulier, le génome de baculovirus déficient pour la réplication est obtenu à partir d'un génome de baculovirus sélectionné parmi ou dérivé du génome de BmNPV, AcMNPV, ApNPV, BsSNPV, CfMNPV, EoSNPV, HaNPV, HzNPV, LdMNPV, MbMNPV, OpMNPV, SIMNPV, SeMNPV ou TeNPV, de préférence AcMNPV.

La présente description concerne également une cellule comprenant un baculovirus recombinant ou une cellule comprenant un jeu d'éléments de recombinaison homologue.

Dans un mode de réalisation particulier, la cellule est une cellule d'insecte, de préférence sélectionnée parmi Sf9, Sf21, Tn5-b14, les lignées cellulaires de lépidoptères sensibles au baculovirus AcMNPV, les lignées Sf21, de préférence Sf9, comme décrit plus en détail dans la partie « préparation du baculovirus recombinant » ci-dessus.

## Procédé de production de polypeptides exogènes

La présente description concerne également un procédé de production de n polypeptides exogènes codés par n gènes exogènes comprenant les étapes de :

a) Fournir un génome de baculovirus déficient pour la réplication dans lequel n gènes essentiels à la réplication virale sont non-fonctionnels;

b) Fournir n vecteurs de transfert comprenant chacun:

  i) une séquence nucléotidique permettant de restaurer la fonction d'un des n gènes essentiels à la réplication virale non-fonctionnel,

  ii) un des n gènes exogènes codant un des n polypeptides, avantageusement une cassette d'expression d'un des n gènes exogènes codant un des n polypeptides,

l'ensemble des séquences d'acides nucléiques i) des n vecteurs étant capables de restaurer la réplication du génome de baculovirus déficient pour la réplication ;

c) Laisser le génome de baculovirus déficient pour la réplication et les n vecteurs de transfert se recombiner dans une cellule d'insecte afin de générer une cellule d'insecte comprenant un génome de baculovirus recombinant capable de se répliquer et capable de générer des baculovirus recombinants comprenant n gènes exogènes ; et

d) Cultiver la cellule d'insecte dans des conditions adaptées pour produire les n polypeptides exogènes ;

n étant un nombre entier au moins égal à 2.

Sauf indication contraire, les définitions et modes de réalisation décrits ci-dessus, notamment dans la partie « préparation du baculovirus recombinant », s'appliquent également à la partie « procédé de production de polypeptides exogènes ».

Dans un mode de réalisation particulier, les gènes essentiels à la réplication virale sont choisis parmi 1629 (ORF9), Pk1 (ORF10), lef-1 (ORF14), ORF34, lef-11 (ORF37), p47 (ORF40), lef8 (ORF50), DNAJ domain (ORF51), ORF53, vp1054 (ORF54), Lef-9 (ORF62), DNA Pol (ORF65), lef-3 (ORF67), ORF73, ORF75, ORF81, p95 (ORF83), vp39 (ORF89), lef-4 (ORF90), p33 (ORF92), helicase (ORF95), vp80 (ORF104), ORF106-107, odv-ec43 (ORF109), gp64/67 (ORF128), ORF132, ORF133, odv-ec27 (ORF144), ORF146, ie1 (ORF147), lef-2 (ORF6), comme détaillé ci-dessus dans la partie « préparation du baculovirus recombinant ».

Avantageusement, dans ledit génome de baculovirus déficient pour la réplication, les n gènes essentiels à la réplication virale non-fonctionnels sont chacun adjacents à un gène non-essentiel à la réplication virale, comme détaillé dans la partie « préparation du baculovirus recombinant » ci-dessus.

Dans un mode de réalisation particulier, le gène non-essentiel à la réplication virale est sélectionné parmi ceux détaillés ci-dessus dans la partie « préparation du baculovirus recombinant ».

Avantageusement, les n gènes exogènes recombinent chacun au niveau d'un gène non-essentiel à la réplication virale adjacent à un gène essentiel à la réplication virale non-fonctionnel, comme détaillé dans la partie « préparation du baculovirus recombinant » ci-dessus.

Dans un mode de réalisation particulier, les vecteurs de transfert comprennent chacun, de part et d'autre de la cassette d'expression du gène exogène, des séquences flanquantes homologues au génome de baculovirus déficient pour la réplication. Avantageusement, les séquences flanquantes de chacun des vecteurs de transfert sont homologues à tout ou partie dudit gène essentiels à la réplication virale non-fonctionnel et à tout ou partie dudit gène non-essentiel à la réplication virale. Avantageusement, les séquences flanquantes ont une longueur qui peut aller de 10 pb (i.e. 10 paires de bases) à 10 kb (i.e. 10 000 paires de bases), avantageusement allant de 100 pb à 6 kb, de préférence allant de 200 pb à 6 kb et, de manière tout à fait préférée allant de 400 pb à 6 kb. Les séquences flanquantes sont décrites en détail dans la partie « préparation du baculovirus recombinant » ci-dessus.

Les n gènes essentiels à la réplication virale sont détaillés dans la partie « préparation du baculovirus recombinant » ci-dessus.

Dans un mode de réalisation particulier, le génome de baculovirus déficient pour la réplication est obtenu à partir d'un génome de baculovirus sélectionné parmi ou dérivé du génome de baculovirus BmNPV, AcMNPV, ApNPV, BsSNPV, CfMNPV, EoSNPV, HaNPV, HzNPV, LdMNPV, MbMNPV, OpMNPV, SIMNPV, SeMNPV ou TeNPV, de préférence AcMNPV., comme détaillé dans la partie « préparation du baculovirus recombinant » ci-dessus.

Dans un mode de réalisation particulier, les n polypeptides exogènes produits par le procédé décrit forment plusieurs protéines. Il peut s'agit de protéines comprenant une seule chaine polypeptidique ou plusieurs sous-unités distinctes. Le nombre de protéines produites peut être égal ou inférieur à n. Par exemple, 3 polypeptides exogènes (n=3) peuvent former (i) une protéine comprenant une seule chaine polypeptidique et une protéine comprenant deux sous-unités, ou (ii) trois protéines comprenant une seule chaine polypeptidique.

Dans un autre mode de réalisation particulier, les n polypeptides exogènes produits par le procédé décrit forment une seule protéine. Dans ce mode de réalisation, la protéine comprend généralement n sous-unités distinctes. Dans un mode de réalisation particulier la protéine est un anticorps monoclonal, une VLP composée de 3 protéines distinctes, un couple de protéines virales, une hormone peptidique, un anticorps bispécifique, un ensemble de trois protéines virales, un complexe multienzymatique, un complexe protéique.

Dans un mode de réalisation particulier, la cellule d'insecte est sélectionnée parmi Sf9, Sf21, Tn5-b14, les lignées cellulaires de lépidoptères sensibles au baculovirus AcMNPV, les lignées Sf21, de préférence Sf9, comme détaillé dans la partie « préparation du baculovirus recombinant » ci-dessus.

Dans un mode de réalisation particulier, le génome de baculovirus déficient pour la réplication est obtenu à partir d'un génome de baculovirus sélectionné parmi ou dérivé du génome de baculovirus BmNPV, AcMNPV, ApNPV, BsSNPV, CfMNPV, EoSNPV, HaNPV, HzNPV, LdMNPV, MbMNPV, OpMNPV, SIMNPV, SeMNPV ou TeNPV, de préférence AcMNPV, comme détaillé dans la partie « préparation du baculovirus recombinant » ci-dessus.

Dans un mode de réalisation particulier, le génome de baculovirus déficient pour la réplication comprend au moins une séquence nucléotidique qui lui permet de se répliquer au sein d'une cellule bactérienne, comme détaillé dans la partie « préparation du baculovirus recombinant » ci-dessus.

Dans un mode de réalisation particulier, le baculovirus recombinant capable de réplication ne comprend pas de séquence nucléotidique qui lui permet de se répliquer au sein d'une cellule bactérienne, comme détaillé dans la partie « préparation du baculovirus recombinant » ci-dessus.

On entend par « conditions adaptées pour produire les polypeptides exogènes » les conditions dans lesquelles les cellules d'insectes sont cultivées pour produire les polypeptides exogènes, notamment un milieu de culture adapté à la croissance optimale des cellules, une température et un pH optimaux. Le milieu de culture peut contenir un sérum d'origine animale.

Dans un mode de réalisation particulier, le procédé de production décrit comprend en outre une étape b') introduire le génome de baculovirus déficient pour la réplication et les n vecteurs de transfert dans une cellule d'insecte. Comme détaillé ci-dessus, l'introduction des vecteurs dans une cellule d'insecte est réalisée selon les techniques générales largement décrites dans l'art antérieur. On peut citer notamment la technique au phosphate de calcium, la technique au DEAE dextran, l'électroporation, les méthodes basées sur les chocs osmotiques, la microinjection ou les méthodes basées sur l'emploi de liposomes, de préférence la lipofection. Dans le cadre de la présente description, le génome de baculovirus déficient pour la réplication et les vecteurs de transfert sont introduits en même temps dans la cellule d'insecte, c'est-à-dire simultanément dans la cellule d'insecte, en d'autre terme le génome de baculovirus déficient pour la réplication et les vecteurs de transfert sont introduits dans la cellule d'insecte en une seule étape. Avantageusement, le génome de baculovirus déficient pour la réplication est introduit dans la cellule d'insecte sous forme circulaire, c'est-à-dire qu'il n'est pas linéarisé au préalable. Comme détaillé plus haut, l'introduction sous forme circulaire est un autre avantage majeur du procédé de production décrit.

La présente description concerne également l'utilisation d'un baculovirus recombinant obtenu selon le procédé selon l'invention ou d'une cellule telle que décrite pour la production de n polypeptides exogènes codés par n gènes exogènes.

## Figures

La **figure 1** est un schéma qui illustre les étapes de préparation du BacMid 1.
Légende:

ORF : open reading frame
Polyédrine ou PH: gène baculovirus codant la polyédrine : gène non essentiel à la réplication virale.
ORF603 : gène baculovirus codant la protéine 603, gène non essentiel.
ORF1629 : gène baculovirus codant la protéine 1629: gène essentiel à la réplication virale pVT : plasmide vecteur de transfert.
Fragment de recombinaison : fragment d'ADN contenant la cassette d'expression à intégrer dans l'ADN cible. Ce fragment présente des régions flanquantes de part et d'autre de la cassette d'expression pour pouvoir cibler spécifiquement la région qui subira la recombinaison homologue dans la bactérie via la Red recombinase.
Mini-F : origine de réplication bactérienne.
$Kan^R$ : cassette d'expression bactérienne exprimant le gène de résistance à la kanamycine.
$Amp^R$ : cassette d'expression bactérienne exprimant le gène de résistance à l'ampicilline.

La **figure 2** est un schéma qui illustre l'étape de délétion partielle du gène codant l'ADN polymérase virale, le gène DNAPol pour la préparation du BacMid 2.
Légende :

gp37 : gène baculovirus codant la glycoprotéine gp37, gène non essentiel à la réplication virale.
DNAPol : gène baculovirus codant l'ADN polymérase virale, gène essentiel à la réplication virale.
DNAPol•Δ466 aa : gène DNAPol délété de la région codant les 466 acides aminés C terminaux
Fragment de recombinaison : fragment d'ADN contenant la cassette d'expression à intégrer dans l'ADN cible. Ce fragment présente des régions flanquantes de part et d'autre de la cassette d'expression pour pouvoir cibler spécifiquement la région qui subira la recombinaison homologue dans la bactérie via la Red recombinase.
$Hygro^R$ : cassette d'expression bactérienne exprimant le gène de résistance à la hygromycine.

La **figure 3** est un schéma qui illustre l'étape de délétion partielle du gène codant la gp64 pour la préparation du BacMid 3.
Légende :

Chit : gène baculovirus codant la chitinase, gène non essentiel à la réplication virale.
Cath : gène baculovirus codant la cathepsine virale, gène non essentiel à la réplication virale.
gp64 : gène baculovirus codant la glycoprotéine gp64 virale, gène essentiel à la réplication virale.
gp64•Δ188 aa : gène gp64 délété de la région codant les 188 acides aminés C terminaux. Fragment de recombinaison : fragment d'ADN contenant la cassette d'expression à intégrer dans l'ADN cible. Ce fragment présente des régions flanquantes de part et d'autre de la cassette d'expression pour pouvoir cibler spécifiquement la région qui subira la recombinaison homologue dans la bactérie via la Red recombinase.
$Zeo^R$ : cassette d'expression bactérienne exprimant le gène de résistance à la zéocine.

La **figure 4** est un schéma qui illustre le vecteur de transfert pVT/gp37 et son utilisation pour l'expression d'un gène X (où X est un gène différent du gène codant la chaine lourde d'un anticorps).
Légende:

pVT : plasmide vecteur de transfert.
gp37 : gène baculovirus codant la glycoprotéine gp37, gène non essentiel à la réplication virale.
DNAPol : gène baculovirus codant l'ADN polymérase virale, gène essentiel à la réplication virale.
DNAPol•Δ466 aa : gène DNAPol délété de la région codant les 466 acides aminés C terminaux.
Gène exogène : gène d'intérêt.
Pr : promoteur viral ou cellulaire qui contrôle l'expression du gène exogène.
$Hygro^R$ : cassette d'expression bactérienne exprimant le gène de résistance à la hygromycine.

La **figure 5** est un schéma qui illustre la construction et l'utilisation du vecteur de transfert PH pVT/PH pour l'expression d'un gène exogène X (où X est un gène exogène différent du gène codant une chaine légère d'un anticorps).

Légende:

pVT/PH : plasmide vecteur de transfert polyédrine.
PH : tout ou partie du gène baculovirus codant la polyédrine, gène non essentiel à la réplication virale.
ORF603: gène baculovirus codant la protéine 603.
ORF1629 : gène baculovirus codant la protéine 1629, gène essentiel à la réplication virale. Gène exogène : gène d'intérêt.
Pr : promoteur viral ou cellulaire qui contrôle l'expression du gène exogène.
$Kan^R$ : cassette d'expression bactérienne exprimant le gène de résistance à la kanamycine.
Mini-F : origine de réplication bactérienne.

La **figure 6** est un schéma qui illustre la construction et l'utilisation du vecteur de transfert pVT/gp37Cγ• pour l'expression de la chaine lourde d'un anticorps.
Légende :

pVT : plasmide vecteur de transfert.
pVT/gp37-Cγ1: plasmide vecteur de transfert spécifique de la chaine lourde d'une immunoglobuline.
Cγ1 : ADNc codant le domaine constant γ1 d'une immunoglobuline humaine.
VH : ADNc codant le domaine variable de la chaine lourde d'une immunoglobuline.
DNAPol : gène baculopvirus codant l'ADN polymérase virale, gène essentiel à la réplication virale.
DNAPolΔ466 aa : gène DNAPol délété de la région codant les 466 acides aminés C terminaux.
Gène exogène : gène d'intérêt.
Pr : promoteur viral ou cellulaire qui contrôle l'expression du gène exogène.
$Hygro^R$ : cassette d'expression bactérienne exprimant le gène de résistance à la hygromycine.
PS : ADNc codant une séquence signal (sécrétion de la chaîne lourde).

La **figure 7** est un schéma qui illustre la construction et l'utilisation du vecteur de transfert pVT/PHCκ pour l'expression de la chaine légère d'un anticorps.
Légende :

ORF603 : gène baculovirus codant la protéine 603.
ORF1629 : gène baculovirus codant la protéine 1629: gène essentiel à la réplication virale. Mini-F : origine de réplication bactérienne.
pVT : plasmide vecteur de transfert.
pVT/PH-Cκ : plasmide vecteur de transfert spécifique de la chaine légère d'une immunoglobuline.
Cκ : ADNc codant le domaine constant • d'une immunoglobuline humaine.
VL : ADNc codant le domaine variable de la chaine légère d'une immunoglobuline.
Pr : promoteur viral ou cellulaire qui contrôle l'expression du gène exogène.
$Kan^R$ : cassette d'expression bactérienne exprimant le gène de résistance à la kanamycine.
PS : ADNc codant une séquence signal (sécrétion de la chaîne légère).

La **figure 8** est un schéma qui illustre la construction du vecteur pVT/Chit-Cath et son utilisation pour la production d'un gène exogène.
Légende :

pVT : Plasmide vecteur de transfert
pVT Chit/cath : Plasmide vecteur de transfert qui cible spécifiquement la région Chit/Cath. Chit : gène baculovirus codant le chitinase non essentiel à la réplication virale
Cath : gène baculovirus codant la cathepsine non essentiel à la réplication virale
Gène exogène : gène d'intérêt.
Pr : promoteur viral ou cellulaire qui contrôle l'expression du gène exogène.
gp64 : gène baculovirus codant la gp64, gène essentiel à la réplication virale
gp64Δ188aa : gène codant la gp64 délétée de 566 bp en 3', ce qui conduit à la production d'une gp64 non fonctionnelle présentant une délétion des 188 acides aminés C-terminaux.
$Zeo^R$ : cassette d'expression bactérienne exprimant le gène de résistance à la zéocine.

La **figure 9** est A) l'analyse par Southern blot du génome de 3 baculovirus recombinants indépendants purifiés générés au cours de la même transfection, et B) l'analyse de l'anticorps recombinant après purification sur protéine A.

Légende :

A : analyse de l'organisation des génomes de 3 baculovirus recombinants indépendants exprimant l'anticorps 13B8II. Ces baculovirus ont été isolés à partir d'une seule expérience de transfection. Les hybridations réalisées respectivement avec une sonde spécifique de la région constante de la chaîne légère kappa : sonde Cκ et une sonde spécifique de la région constante de la chaine lourde gamma 1 : sonde Cγ1 démontrent une organisation correcte et identique des 3 virus recombinants.
B. Analyse par électrophorèse en gel de polyacrylamide (SDS, 2-mercaptoéthanol) et coloration argentique de l'anticorps recombinant purifié. L'anticorps sécrété dans le milieu de culture des cellules infectées par le baculovirus recombinant a été purifié sur colonne de Protéine A Sepharose.
PC1 : Plasmide contrôle, plasmide contenant le gène de la chaine légère kappa, PC2 : Plasmide contrôle, plasmide contenant le gène de la chaine lourde γ1, R1-3, baculovirus recombinant 1, 2 et 3, MW : marqueur de taille.

La **figure 10** est l'analyse en Southern blot du génome d'un virus triple recombinant exprimant les protéines M, HA et NA du virus de la grippe. Légende :

M : gène du virus de la grippe codant la protéine de matrice.
HA : gène du virus de la grippe codant l'hémagglutinine.
NA : gène du virus de la grippe codant la neuraminidase.
bp : taille des fragments d'ADN exprimée en paire de base.

La **figure 11** est un schéma qui illustre en A la structure de l'anticorps bispécifique en B l'analyse par électrophorèse en gel de polyacrylamide de l'anticorps bispécifique purifié sur colonne de protéine A Sepharose.
Légende :

A : Représentation schématique de la structure de l'anticorps bispécifique. L1 : chaine légère de l'anticorps 1 ; L2, chaine légère de l'anticorps 2.
B : Anticorps bispécifique purifié, analysé par électrophorèse sur gel de polyacrylamide. Les protéines sont révélées par coloration argentique. (1) électrophorèse en conditions réductrices (SDS, 2-mercaptoéthenol). (2) électrophorèse en conditions non réductrices. H : chaine lourde de l'anticorps, L : chaîne légère de l'anticorps, H2L4 : composition de l'anticorps bispécifique : 2 chaînes lourdes fusionnées reliées par 4 ponts disulfures au niveau de 2 régions charnières + 4 chaines légères (2 chaines L1 + 2 chaines L2) appariées de manière spécifique aux régions VH1-CH1 et VH2-CH1 correspondantes.

## Exemples

### Exemple 1: Construction d'un génome de baculovirus déficient pour la réplication dans lequel 1 gène essentiel à la réplication virale est non-fonctionnel (BacMid 1)

Le BacMid 1 présente la délétion d'un gène essentiel à la réplication virale, le gène 1629.

*1.1. Intégration de l'origine de réplication bactérienne dans un génome du baculovirus*

Cette opération est réalisée dans la cellule d'insecte.

L'origine de réplication bactérienne Mini-F a été introduite dans le locus polyédrine du génome de baculovirus AcMNPV par recombinaison homologue dans les cellules d'insectes Sf9 (*Spodoptera frugiperda*). Pour cela, les cellules ont été transfectées avec (i) un vecteur de transfert PH (pVT/Mini-F $Kan^R$) dans lequel la séquence du gène ph a été remplacée par un fragment d'ADN portant le Mini-F + une cassette d'expression bactérienne conférant la résistance à la Kanamycine ($Kan^R$), et (ii) un génome de baculovirus AcMNPV (Baculovirus isolé à partir du lépidoptère *Autographa californica).* Les baculovirus générés ont été purifiés par la technique des plages de lyse puis caractérisés afin de confirmer qu'ils avaient bien intégré le Mini-F et la cassette d'expression $Kan^R$. Un baculovirus a été sélectionné et a ensuite été transféré dans la bactérie *E. coli* EL350, générant ainsi un premier BacMid (BacMid 0, non déficient pour la réplication virale en cellules d'insecte).

*1.2. Délétion du gène essentiel 1629*

Une cassette d'expression bactérienne conférant la résistance à l'ampicilline ($Amp^R$) et présentant en 5' et 3' le site de restriction MauBI - site absent du génome de baculovirus *Ac*MNPV - a été intégré en aval de la cassette d'expression bactérienne $Kan^R$ par recombinaison homologue dans la bactérie *E. coli* EL350. Au cours de cette recombinaison, un fragment d'ADN codant les 27 acides aminés C-terminaux de la protéine 1629 a été délété, rendant la protéine 1629 non-fonctionnelle. Le gène de résistance à l'ampicilline a ensuite été éliminé après digestion par MauBI puis religation, générant ainsi le BacMid 1. Le génome du baculovirus (i.e. le BacMid 1) est alors déficient pour la réplication en cellules d'insectes, car un gène essentiel à la réplication virale (i.e. le gène codant la protéine 1629) est non-fonctionnel. Les bactéries contenant le BacMid 1 sont appelées ci-après « bactéries *E. coli* EL350/BacMid1 ».
La figure 1 illustre les étapes de préparation du BacMid 1.

**Exemple 2 : Construction d'un génome de baculovirus déficient pour la réplication dans lequel 2 gènes essentiels à la réplication virale sont non-fonctionnels (BacMid 2)**

Le BacMid 2 présente la délétion de 2 gènes essentiels à la réplication virale, le gène 1629 et le gène codant l'ADN polymérase virale (DNAPol). A partir du BacMid 1, la délétion du gène DNAPol a été réalisée dans les bactéries *E. coli* EL350/BacMid1 après électroporation d'un fragment de recombinaison de 4222 bp dans lequel une partie des gènes codant la gp37 (240 acides aminés) et la DNAPol (466 acides aminés C-terminaux) a été délétée et remplacée par une cassette d'expression bactérienne permettant la production d'hygromycine B phosphotransférase ($Hygro^R$) conférant ainsi la résistance à l'hygromycine ($Hygro^R$). Le gène $Hygro^R$ a été placé sous le contrôle du promoteur bactérien EM7 (issu du vecteur commercial pSelect-Hygro-mcs, Invitrogen), le terminateur glms a été introduit en aval du gène $Hygro^R$ (Gay N.J. et al. Biochem J., 1986, 234, 111-117). Les bactéries contenant le BacMid2 (*E. coli* EL350/BacMid2) ont été sélectionnées pour leur résistance à la l'hygromycine. Le génome de baculovirus (i.e. le BacMid 2) est déficient pour la réplication en cellules d'insectes, car deux gènes essentiels à la réplication virale (i.e. le gène codant la protéine 1689 et le gène codant DNAPol) sont non-fonctionnels.
La **figure 2** est un schéma qui illustre l'étape de délétion de DNAPol pour la préparation du BacMid 2.
**Note :** On peut utiliser le BacMid 2 pour produire une seule protéine (voir exemple 4). Il suffit d'avoir deux vecteurs de transfert, l'un apportant le gène exogène à produire et tout ou partie du gène délété 1 et l'autre apportant le gène sauvage correspondant au gène délété 2. Les deux gènes délétés sont réparés lors de la recombinaison homologue.

**Exemple 3 : Construction d'un génome de baculovirus déficient pour la réplication dans lequel 3 gènes essentiels à la réplication virale sont non-fonctionnels (BacMid 3)**

Le BacMid 3 présente la délétion de 3 gènes essentiels, 1629, DNAPol et gp64.
A partir du BacMid 2, la délétion du gène gp64 a été effectuée dans les bactéries *E. coli* EL350/BacMid2 après électroporation d'un fragment de recombinaison de 3260pb dans lequel la totalité du gène de la cathepsine plus 779 bp de la séquence codant 259 aa de la chitinase et une partie du gène de la gp64, délétion de 566 bp codant 188 acides aminés, a été remplacée par une cassette d'expression bactérienne conférant une résistance à la Zéocine (Drocourt et al., Nucleic Acids Research, vol.18n°13, 1990). Le gène Zéocine® issu du plasmide commercial pCR®-Blunt (Invitrogen) a été placé sous contrôle du promoteur bactérien T5N25, issu du phage T5 (Gentz and Bujard, J. Bacteriology, vol.164n°1, 1985*)* et suivi du terminateur de transcription rrnBT1 (*E.coli* ribosomal RNA opéron T1 terminator) (Kwon et al., J Biol. Chem., vol 274 n°41, 1999*)*. Les bactéries contenant le BacMid3 (*E. coli* EL350/BacMid3) ont été sélectionnées pour leur résistance à la Zéocine. Le génome de baculovirus (i.e. le BacMid 3) est déficient pour la réplication en cellules d'insectes, car trois gènes essentiels à la réplication virale (i.e. le gène codant la protéine 1689 et le gène codant DNAPol et le gène codant gp64) sont non-fonctionnels.
La **figure 3** est un schéma qui illustre l'étape de délétion de gp64 pour la préparation du BacMid 3.

**Exemple 4 : Utilisation du BacMid 2**

Un vecteur de transfert pVT/gp37 a été construit pour pouvoir générer des baculovirus recombinants exprimant 2 gènes exogènes. Pour cela, le fragment EcoRI F du génome de baculovirus AcMNPV contenant le gène gp37 et le gène DNAPol a été cloné dans un plasmide bactérien pUC, générant ainsi le pUC/gp37.
Ce plasmide a ensuite été modifié de la façon suivante : une grande partie du gène codant la gp37 a été délété (724pb), l'ATG initiateur a été muté et remplacé par deux sites de restriction uniques XbaI et Avril permettant l'intégration d'un gène exogène sous contrôle du promoteur naturel de la gp37. Ces modifications ont ainsi conduit à l'obtention du vecteur de transfert pVT/gp37.

Les cellules Sf9 ont été transfectées par lipofection avec les vecteurs de transfert pVT/PH et pVT/gp37 chargés avec les gènes exogènes et l'ADN du BacMid 2. Les virus générés après la recombinaison homologue ont été clonés par la méthode des plages de lyse. La production de la protéine recombinante a été vérifiée par une méthode adaptée (e.g. par exemple ELISA, Western blot, dosage enzymatique). Le génome des virus recombinants a été vérifié par Southern blot et la séquence de l'exogène intégré dans le génome viral a été vérifiée par séquençage après amplification PCR.

La **figure 4** est un schéma qui illustre le vecteur de transfert pVT/gp37 pour l'expression d'un gène X (où X est un gène différent du gène codant la chaine lourde d'un anticorps).

Les génomes de baculovirus recombinant générés après recombinaison homologue entre le BacMid2 et les vecteurs de transfert n'expriment plus la gp37 (protéine non-essentielle à la réplication virale).

Pour que l'ADN viral soit réparé dans les 2 locus, du BacMid 2, une seconde recombinaison doit avoir lieu avec un vecteur de transfert PH chargé ou non avec un gène exogène. Dans tous les cas, l'ADN du génome de baculovirus sera réparé et donc infectieux.

Il sera possible également d'utiliser le pVT/PH contenant une séquence sauvage, c'est-à-dire contenant la cassette d'expression sauvage (non modifiée) conduisant à la production de polyédrine. Le pVT/PH pourra également être « vide » c'est-à-dire ne pas contenir de gène exogène ni le gène polyédrine.

La **figure 5** est un schéma qui illustre la construction et l'utilisation du vecteur de transfert PH pVT/PH pour l'expression d'un gène exogène X (où X est un gène exogène différent du gène codant une chaine légère d'un anticorps).

<u>Expression de la chaine lourde d'un anticorps.</u>

Construction d'un pVT/gp37 spécifique pVT/gp37-C$\gamma$1

Ce vecteur de transfert contient la cassette d'expression suivante :

- Promoteur viral P10 sauvage (SEQ ID NIO :1)
- Séquence d'ADN codant une séquence signal d'une immunoglobuline humaine (séquence de sécrétion)
- 2 sites de restriction uniques pour le clonage en phase de la région variable (VH) de l'anticorps (région qui donne la spécificité de l'anticorps)
- Séquence d'ADN qui code une région constante d'IgG ($\gamma$1-4) epsilon, mu, ou alpha humaine.

La **figure 6** est un schéma qui illustre la construction et l'utilisation du vecteur de transfert pVT/gp37C$\gamma$1 pour l'expression de la chaine lourde d'un anticorps.

<u>Expression de la chaine légère d'un anticorps.</u>

Construction d'un pVT/PH-VL spécifique. Ce vecteur de transfert contient la cassette d'expression suivante :

- Promoteur viral P10 P10S1B (SEQ ID NO :2)
- Séquence d'ADN codant une séquence signal d'une immunoglobuline humaine (séquence de sécrétion)
- 2 sites de restriction uniques pour le clonage en phase de la région variable (VL) de l'anticorps (région qui donne la spécificité de l'anticorps)
- Séquence d'ADN qui code une région constante de chaine légère kappa ($\kappa$) ou lambda (l) d'IgG humaine.

La **figure 7** est un schéma qui illustre la construction et l'utilisation du vecteur de transfert pVT/PHC• pour l'expression de la chaine légère d'un anticorps.

**Exemple 5 : Utilisation du BacMid 3.**

Un vecteur de transfert, pVT/Chit-Cath a été construit pour pouvoir générer des génomes de baculovirus recombinants exprimant 3 gènes exogènes.

Le fragment BstXI-XbaI issu des régions EcoRI E et H du baculovirus AcMNPV a été cloné dans un plasmide pUC. Une délétion EcoNI-EcoRI de 1175 pb permet d'inactiver les gènes codant la chitinase (*chiA*), non essentielle in vitro et la cathepsine (*v-cath*) non essentielle également. L'addition d'un site XbaI entre les sites EcoNI et EcoRI permet d'intégrer un gène exogène. Ces modifications ont ainsi conduit à l'obtention du vecteur de transfert pVT/Chit-Cath.

Les cellules Sf9 sont transfectées par lipofection avec les vecteurs de transfert pVT/PH, pVT/gp37 et pVT/chit-Cath chargés avec les gènes exogènes et l'ADN du BacMid 3. Les virus générés au cours de la recombinaison homologue ont été clonés par la méthode des plages de lyse. La production de la protéine recombinante a été contrôlée par une méthode adaptée, ELISA, Western blot, dosage enzymatique ... le génome des virus recombinants a été contrôlé par Southern blot et la séquence du gène exogène a été contrôlée après amplification PCR.

La **figure 8** est un schéma qui illustre la construction du vecteur pVT/Chit-Cath et sa recombinaison homologue avec le BacMid 3.

**Exemple 6 : Production d'un anticorps monoclonal *anti-CD4 (13B8II)* en utilisant le BacMid 2.**

Les ADNc codant les régions VH et VL de l'anticorps ont été intégrées respectivement dans les vecteurs de transfert pVT/PH-C• et pVT/gp37-Cγ1. Des baculovirus recombinants ont été générés après recombinaison homologue entre les 2 pVT et l'ADN du BacMid 2 de l'exemple 4 :

L'ADNc codant la région VL de l'anticorps a été introduite dans le pVTPH/Ck qui recombine avec la région PH/1629 du BacMid2,
L'ADNc codant la région VH de l'anticorps a été clonée dans le pVT/gp37-Cγ1 qui recombine avec la région gp37 du BacMid2.

Les cellules Sf9 ont été transfectées par lipofection avec le BacMid 2 et les 2 vecteurs de transfert obtenus dans l'exemple 4 puis incubées pendant 4 jours à 28°C. Les surnageants de culture ont été prélevés et les baculovirus recombinants générés, sécrétés dans le milieu de culture ont été clonés par la technique des plages de lyse.

L'organisation du génome des baculovirus recombinants a été contrôlée par Southern blot (voir figure 9) et les gènes exogènes intégrés (i.e. VL et VH) ont été vérifiés après amplification PCR, clonage puis séquençage.

**Exemple 7 : Utilisation du BacMid 3 pour la production de VLP (Virus-Like-Particle).**

***Production de VLP de grippe***

Pour produire ces VLPs, les 3 gènes du virus de la grippe, M, HA et NA ont été co-exprimés. Ces 3 gènes ont été intégrés dans les trois vecteurs de transfert nécessaires pour recombiner avec le BacMid 3 de l'exemple 5 :

Le gène M a été introduit dans le vecteur de transfert pVT/PH comme décrit dans la figure 5.
Le gène HA a été introduit dans le vecteur de transfert pVT/gp37 comme décrit dans la figure 4.
Le gène NA a été introduit dans le vecteur de transfert pVT/Chit/Cath comme décrit dans la figure 8.

Les cellules Sf9 ont été transfectées par lipofection avec le BacMid 3 et les 3 vecteurs de transfert obtenus ci-dessus, puis incubées pendant 4 jours à 28°C. Les baculovirus recombinants générés puis sécrétés dans le surnageant de culture ont été clonés par la méthode des plages de lyse.

L'organisation des génomes de baculovirus recombinants a été contrôlée par Southern blot (voir figure 10) et les gènes intégrés ont été vérifiés après amplification PCR, clonage puis séquençage. Le Southern blot a été réalisé sur l'ADN génomique du virus recombinant exprimant les 3 protéines HA, NA et M du virus de la grippe pour détecter les l'ADNc codant les protéines M, HA et NA intégrées. Les membranes ont été hybridées avec des sondes spécifiques de ces 3 gènes.

**Exemple 8 : Utilisation du BacMid 3 pour la production d'anticorps bispécifiques.**

L'anticorps bispécifique construit selon le brevet (PCT/IB2012/053482) est constitué d'une chaine lourde composée des domaines VH+CH1+CH2+CH3 d'un anticorps 1, fusionnée en N-terminal aux domaines VH+CH1 d'un anticorps 2. Des mutations introduites à l'interface des régions CL et CH1 de l'anticorps 1 favorisent les appariements corrects entre les domaines VL1 et VL2 des chaines légères L1 et L2 qui sont produites séparément et les domaines VH1 et VH2 correspondants. La production de cet anticorps nécessite la production simultanée et en quantité égale de 3 chaines, la chaine lourde fusionnée, la chaine légère L1 et la chaine légère L2.

L'ADNc codant la chaine légère L1 a été introduit dans le vecteur de transfert pVT/PH comme décrit dans la figure 5.

L'ADNc codant la chaîne légère L2 a été introduit dans le vecteur de transfert pVT/gp37 comme décrit dans la figure 4.

L'ADN c codant la chaine lourde fusionnée a été introduit dans le vecteur de transfert pVT/Chit-Cath comme décrit dans la figure 8.

La **figure 11** est un schéma qui illustre en A la structure de l'anticorps bispécifique en B l'analyse par électrophorèse en gel de polyacrylamide de l'anticorps bispécifique purifié sur colonne de protéine A Sepharose.

## Revendications

1. Procédé pour préparer, dans une cellule d'insecte, un baculovirus recombinant comprenant n gènes d'intérêt, par multirecombinaison homologue entre :

    a) un génome de baculovirus déficient pour la réplication dans lequel n gènes essentiels à la réplication virale sont non-fonctionnels ; et
    b) n vecteurs de transfert comprenant chacun:

        i) une séquence nucléotidique permettant de restaurer la fonction d'un des n gènes essentiels à la réplication virale non-fonctionnel,
        ii) un des n gènes d'intérêt,

    l'ensemble des séquences nucléotidiques i) des n vecteurs de transfert étant capables de restaurer la réplication du génome de baculovirus déficient pour la réplication ;
    n étant un nombre entier au moins égal à 2, et
    le génome de baculovirus déficient pour la réplication et les n vecteurs de transfert étant introduits en une seule étape dans la cellule d'insecte.

2. Procédé selon la revendication 1, dans lequel les gènes essentiels à la réplication virale sont choisis parmi 1629 (ORF9), Pk1 (ORF10), lef-1 (ORF14), ORF34, lef-11 (ORF37), p47 (ORF40), lef8 (ORF50), DNAJ domain (ORF51), ORF53, vp1054 (ORF54), Lef-9 (ORF62), DNA Pol (ORF65), lef-3 (ORF67), ORF73, ORF75, ORF81, p95 (ORF83), vp39 (ORF89), lef-4 (ORF90), p33 (ORF92), helicase (ORF95), vp80 (ORF104), ORF106-107, odv-ec43 (ORF109), gp64/67 (ORF128), ORF132, ORF133, odv-ec27 (ORF144), ORF146, ie1 (ORF147), lef-2 (ORF6).

3. Procédé selon l'une quelconque des revendications 1 ou 2, dans lequel les n gènes essentiels à la réplication virale non-fonctionnels sont chacun adjacents à un gène non-essentiel à la réplication virale.

4. Procédé selon la revendication 3, dans lequel le gène non-essentiel à la réplication virale est sélectionné parmi Ph (ORF 8), ORF11, ORF13, egt (ORF15), v-ubiquitin (ORF35), 39K (ORF36), ORF38, p43 (ORF39), lef-12 (ORF41), pcna (ORF49), ORF52, ORF55, Fp (ORF61), ORF63, gp37 (ORF64), ORF68, ORF72, ORF74, ORF82, cg30 (ORF88), ORF91, pif-4 (ORF96), he65 (ORF105), ORF108, ORF110, cathepsine (ORF127), p24 (ORF129), pp34 (ORF131), ORF134, ORF145, odv-e56 (ORF148), ORF5.

5. Procédé selon l'une quelconque des revendications 3 ou 4, dans lequel les n gènes d'intérêt recombinent chacun au niveau d'un gène non-essentiel à la réplication virale adjacent à un gène essentiel à la réplication virale non-fonctionnel.

6. Procédé selon l'une quelconque des revendications 1 à 5, dans lequel les vecteurs de transfert comprennent, de part et d'autre de la cassette d'expression du gène d'intérêt, des séquences flanquantes homologues au génome de baculovirus déficient pour la réplication.

7. Procédé selon la revendications 6, dans lequel les séquences flanquantes ont une longueur allant de 10 pb (i.e. 10 paires de bases) à 10 kb (i.e. 10 000 paires de bases), avantageusement allant de 100 pb à 6 kb, de préférence allant de 200 pb à 6 kb et, de manière tout à fait préférée allant de 400 pb à 6 kb.

8. Procédé selon l'une quelconque des revendications 1 à 7, dans lequel la cellule d'insecte est sélectionnée parmi Sf9, Sf21, Tn5-b14, les lignées cellulaires de lépidoptères sensibles au baculovirus AcMNPV, les lignées Sf21, de préférence Sf9.

9. Procédé selon l'une quelconque des revendications 1 à 8, dans lequel le génome de baculovirus déficient pour la réplication est obtenu à partir d'un génome de baculovirus sélectionné parmi ou dérivé du génome de BmNPV, AcMNPV, ApNPV, BsSNPV, CfMNPV, EoSNPV, HaNPV, HzNPV, LdMNPV, MbMNPV, OpMNPV, SIMNPV, SeMNPV ou TeNPV, de préférence AcMNPV.

**10.** Procédé selon l'une quelconque des revendications 1 à 9, dans lequel n un nombre entier allant de 2 à 30, par exemple allant de 2 à 10.

## Patentansprüche

**1.** Verfahren zur Herstellung, in einer Insektenzelle, eines rekombinanten Baculovirus, umfassend n interessierende Gene, durch homologe Multirekombination zwischen:

a) einem für die Replikation defizienten Baculovirusgenom, wobei für die virale Replikation n wesentliche Gene nichtfunktional sind; und
b) n Transfervektoren, die jeweils umfassen:

i) eine Nukleotidsequenz, die erlaubt, die Funktion eines der für die virale Replikation n wesentlichen nichtfunktionalen Gene wiederherzustellen,
ii) eins der n interessierende Gene,

wobei die Gesamtheit der Nukleotidsequenzen i) der n Transfervektoren imstande ist, die Replikation des für die Replikation defizienten Baculovirusgenoms wiederherzustellen;
wobei n eine Ganzzahl von mindestens gleich 2 ist, und
das für die Replikation defiziente Bakulovirusgenom und die n Transfervektoren in einem einzigen Schritt in die Insektenzelle eingeführt werden.

**2.** Verfahren nach Anspruch 1, wobei die für die virale Replikation wesentlichen Gene aus 1629 (ORF9), Pk1 (ORF10), lef-1 (ORF14), ORF34, lef-11 (ORF37), p47 (ORF40), lef8 (ORF50), DNAJ Domain (ORF51), ORF53, vp1054 (ORF54), Lef-9 (ORF62), DNA Pol (ORF65), lef-3 (ORF67), ORF73, ORF75, ORF81, p95 (ORF83), vp39 (ORF89), lef-4 (ORF90), p33 (ORF92), Helicase (ORF95), vp80 (ORF104), ORF106-107, odv-ec43 (ORF109), gp64/67 (ORF128), ORF132, ORF133, odv-ec27 (ORF144), ORF146, ie1 (ORF147), lef-2 (ORF6) ausgewählt sind.

**3.** Verfahren nach einem der Ansprüche 1 oder 2, wobei die für virale Replikation n wesentlichen nichtfunktionalen Gene jeweils an ein für die virale Replikation nichtwesentliches Gen angrenzen.

**4.** Verfahren nach Anspruch 3, wobei das für die virale Replikation nichtwesentliche Gen aus Ph (ORF 8), ORF11, ORF13, egt (ORF15), v-Ubiquitin (ORF35), 39K (ORF36), ORF38, p43 (ORF39), lef-12 (ORF41), pcna (ORF49), ORF52, ORF55, Fp (ORF61), ORF63, gp37 (ORF64), ORF68, ORF72, ORF74, ORF82, cg30 (ORF88), ORF91, pif-4 (ORF96), he65 (ORF105), ORF108, ORF110, Cathepsine (ORF127), p24 (ORF129), pp34 (ORF131), ORF134, ORF145, odv-e56 (ORF148), ORF5 ausgewählt ist.

**5.** Verfahren nach einem der Ansprüche 3 oder 4, wobei die n interessierenden Gene jeweils im Bereich eines für die virale Replikation nichtwesentlichen Gens rekombinieren, das an ein für die virale Replikation wesentliches nichtfunktionales Gen angrenzt.

**6.** Verfahren nach einem der Ansprüche 1 bis 5, wobei die Transfervektoren beiderseits der Expressionskassette des interessierenden Gens flankierende Sequenzen umfassen, die zum für die Replikation defizienten Bakulovirusgenom homolog sind.

**7.** Verfahren nach Anspruch 6, wobei die flankierenden Sequenzen eine Länge haben, die von 10 pb (d. h. 10 Basenpaare) bis 10 kb (d. h. 10.000 Basenpaare), in vorteilhafter Weise von 100 pb bis 6 kb, vorzugsweise von 200 pb bis 6 kb und sehr bevorzugt von 400 pb bis 6 kb reicht.

**8.** Verfahren nach einem der Ansprüche 1 bis 7, wobei die Insektenzelle aus Sf9, Sf21, Tn5-b14, den für Baculovirus sensiblen Schmetterlings-Zelllinien AcMNPV, den Linien Sf21, vorzugsweise Sf9, ausgewählt ist.

**9.** Verfahren nach einem der Ansprüche 1 bis 8, wobei das für die Replikation defiziente Bakulovirusgenom aus einem Bakulovirusgenom gewonnen wird, das aus oder von dem Genom von BmNPV, AcMNPV, ApNPV, BsSNPV, Cf-

MNPV, EoSNPV, HaNPV, HzNPV, LdMNPV, MbMNPV, OpMNPV, SIMNPV, SeMNPV oder TeNPV, vorzugsweise AcMNPV, ausgewählt oder abgeleitet ist.

**10.** Verfahren nach einem der Ansprüche 1 bis 9, wobei n eine Ganzzahl von 2 bis 30, beispielsweise von 2 bis 10 ist.

**Claims**

**1.** Method for producing a recombinant baculovirus comprising n genes of interest in an insect cell, by means of multiple homologous recombination between:

a) a replication deficient baculovirus genome in which n genes essential to the viral replication are non-functional; and
b) n transfer vectors each comprising:

i) a nucleotide sequence making it possible to restore the function of one of the n non-functional genes essential to the viral replication,
ii) one of the n genes of interest,

all of the nucleotide sequences i) of the n transfer vectors being capable of restoring the replication of the replication-deficient baculovirus genome;
n being an integer equal to at least 2, and
the replication-deficient baculovirus genome and the n transfer vectors being introduced into the insect cell in a single step.

**2.** Method according to claim 1, wherein the genes essential to the viral replication are chosen from 1629 (ORF9), Pk1 (ORF10), lef-1 (ORF14), ORF34, lef-11 (ORF37), p47 (ORF40), lef8 (ORF50), DNAJ domain (ORF51), ORF53, vp1054 (ORF54), Lef-9 (ORF62), DNA Pol (ORF65), lef-3 (ORF67), ORF73, ORF75, ORF81, p95 (ORF83), vp39 (ORF89), lef-4 (ORF90), p33 (ORF92), helicase (ORF95), vp80 (ORF104), ORF106-107, odv-ec43 (ORF109), gp64/67 (ORF128), ORF132, ORF133, odv-ec27 (ORF144), ORF146, ie1 (ORF147), lef-2 (ORF6).

**3.** Method according to any one of claims 1 or 2, wherein the n non-functional genes essential to the viral replication are each adjacent to a gene that is not essential to the viral replication.

**4.** Method according to claim 3, wherein the gene that is not essential to the viral replication is selected from Ph (ORF 8), ORF11, ORF13, egt (ORF15), v-ubiquitin (ORF35), 39K (ORF36), ORF38, p43 (ORF39), lef-12 (ORF41), pcna (ORF49), ORF52, ORF55, Fp (ORF61), ORF63, gp37 (ORF64), ORF68, ORF72, ORF74, ORF82, cg30 (ORF88), ORF91, pif-4 (ORF96), he65 (ORF105), ORF108, ORF110, cathepsin (ORF127), p24 (ORF129), pp34 (ORF131), ORF134, ORF145, odv-e56 (ORF148), ORF5.

**5.** Method according to any one of claims 3 or 4, wherein the n genes of interest each recombine with a gene that is not essential to the viral replication adjacent to a non-functional gene essential to the viral replication.

**6.** Method according to any one of claims 1 to 5, wherein the transfer vectors comprise, on either side of the expression cassette of the gene of interest, flanking sequences homologous to the replication-deficient baculovirus genome.

**7.** Method according to claim 6, wherein the flanking sequences have a length ranging from 10 bp (i.e. 10 base pairs) to 10 kb (i.e. 10,000 base pairs), advantageously ranging from 100 bp to 6 kb, preferably ranging from 200 bp to 6 kb and, most preferably, ranging from 400 bp to 6 kb.

**8.** Method according to any one of claims 1 to 7, wherein the insect cell is selected from Sf9, Sf21, Tn5-b14, lepidopteran cell lines sensitive to the AcMNPV baculovirus, Sf21 lines, preferably Sf9.

**9.** Method according to any one of claims 1 to 8, wherein the replication-deficient baculovirus genome is obtained from a baculovirus genome selected from, or derived from, the genome of BmNPV, AcMNPV, ApNPV, BsSNPV, CfMNPV, EoSNPV, HaNPV, HzNPV, LdMNPV, MbMNPV, OpMNPV, SIMNPV, SeMNPV or TeNPV, preferably AcMNPV.

**10.** Method according to any one of claims 1 to 9, wherein n is an integer ranging from 2 to 30, for example ranging from 2 to 10.

Figure 1

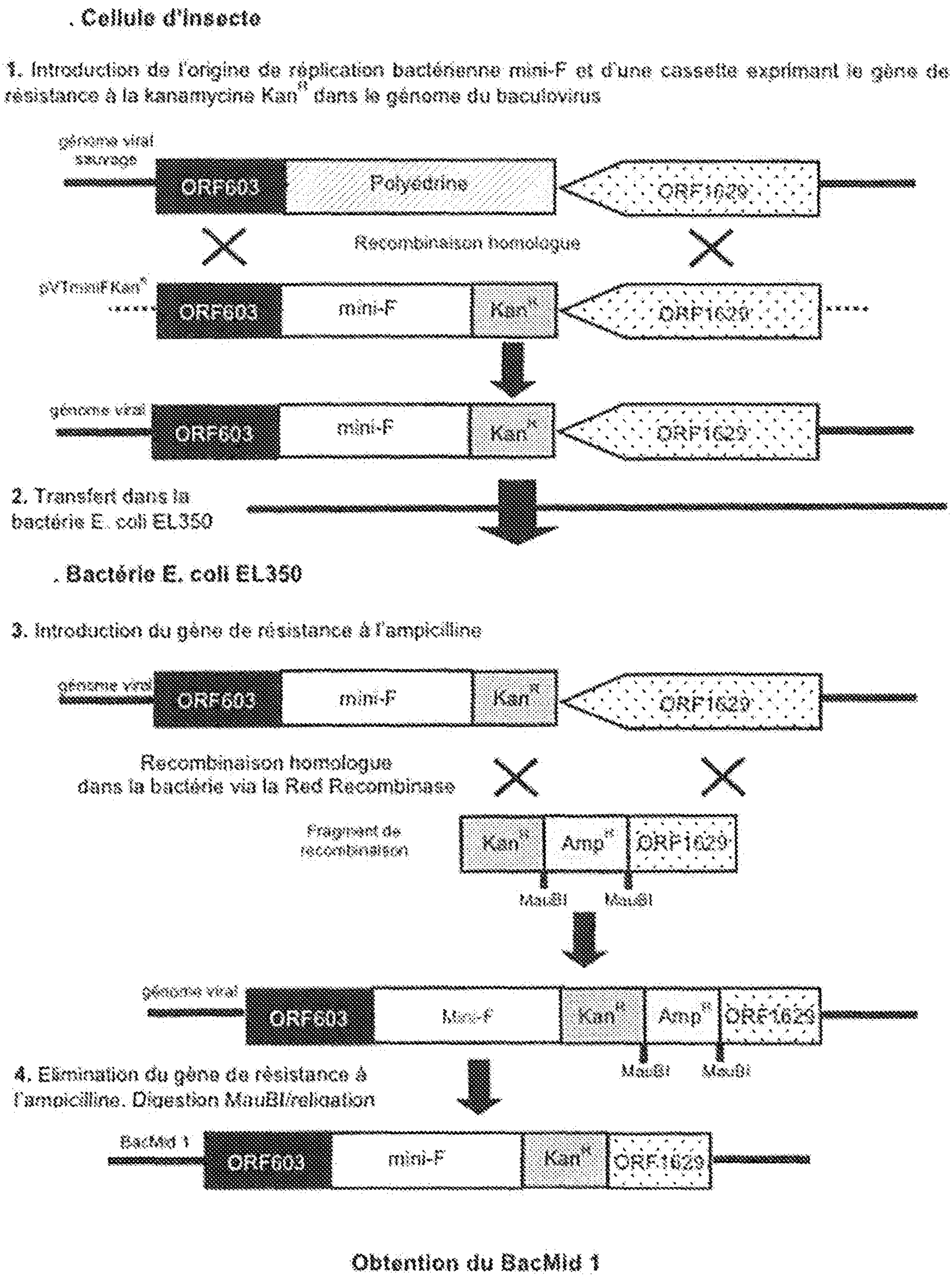
. Cellule d'insecte
1. Introduction de l'origine de réplication bactérienne mini-F et d'une cassette exprimant le gène de résistance à la kanamycine Kan[R] dans le génome du baculovirus
génome viral sauvage
ORF603
Polyédrine
ORF1629
Recombinaison homologue
pVTminiFKan[R]
ORF603
mini-F
Kan[R]
ORF1629
génome viral
ORF603
mini-F
Kan[R]
ORF1629
2. Transfert dans la bactérie E. coli EL350
. Bactérie E. coli EL350
3. Introduction du gène de résistance à l'ampicilline
génome viral
ORF603
mini-F
Kan[R]
ORF1629
Recombinaison homologue dans la bactérie via la Red Recombinase
Fragment de recombinaison
Kan[R]
Amp[R]
ORF1629
MauBI
MauBI
génome viral
ORF603
Mini-F
Kan[R]
Amp[R]
ORF1629
MauBI
MauBI
4. Elimination du gène de résistance à l'ampicilline. Digestion MauBI/religation
BacMid 1
ORF603
mini-F
Kan[R]
ORF1629
Obtention du BacMid 1

Figure 2

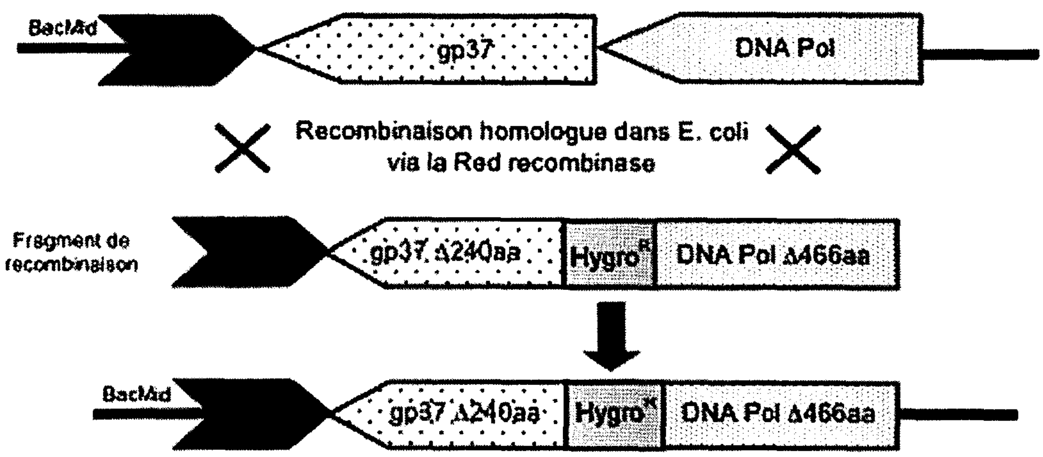


**Obtention du BacMid 2**

Figure 3

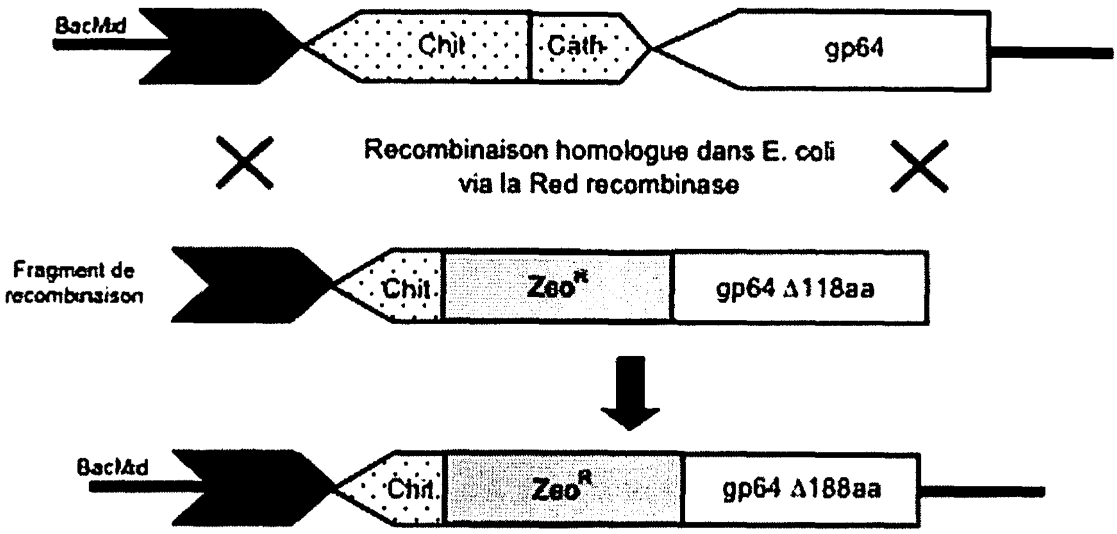


**Obtention du BacMid 3**

Figure 4

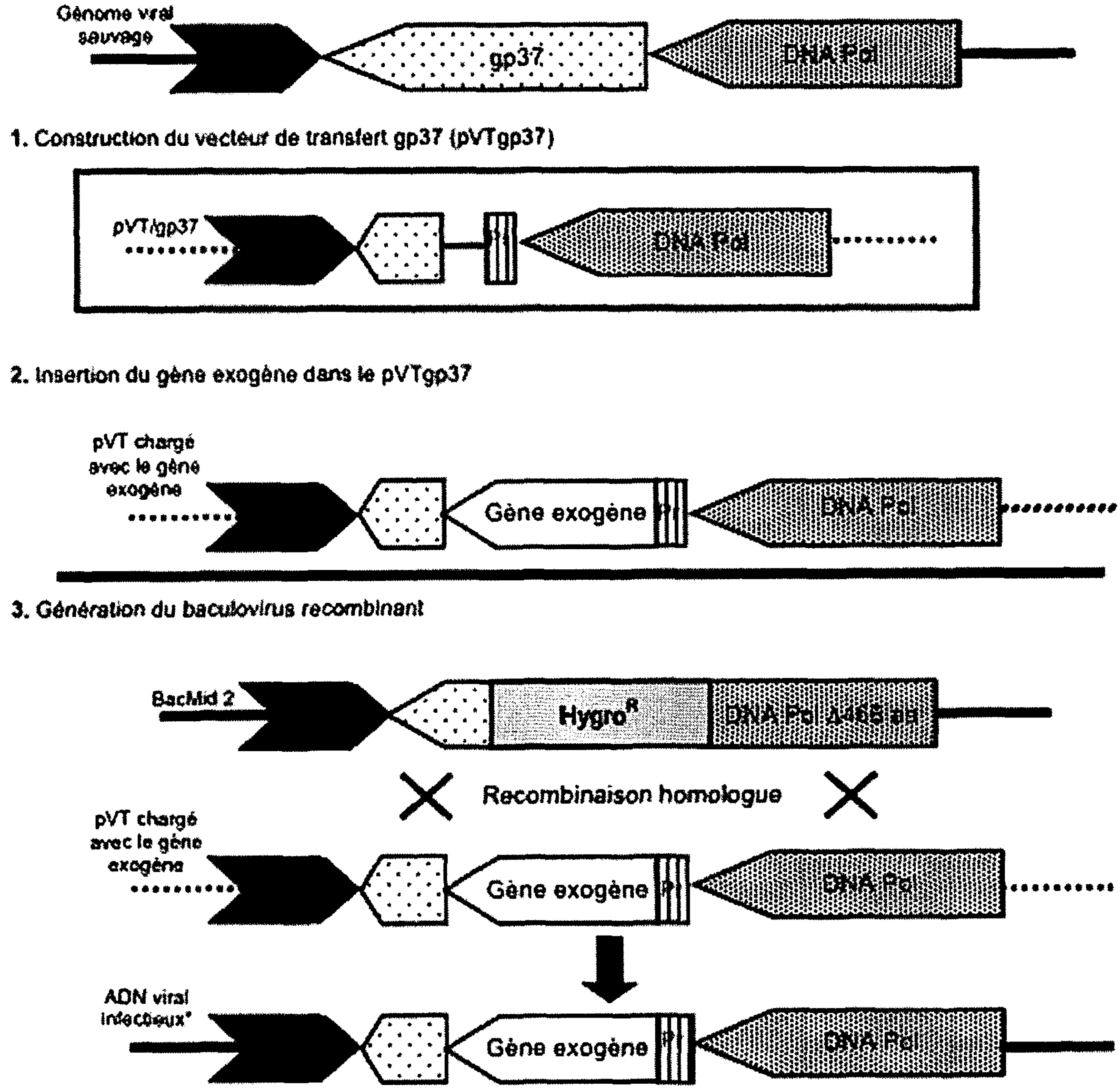


(*) ADN viral infectieux si la recombinaison dans le locus PH s'est produite également.

Figure 5

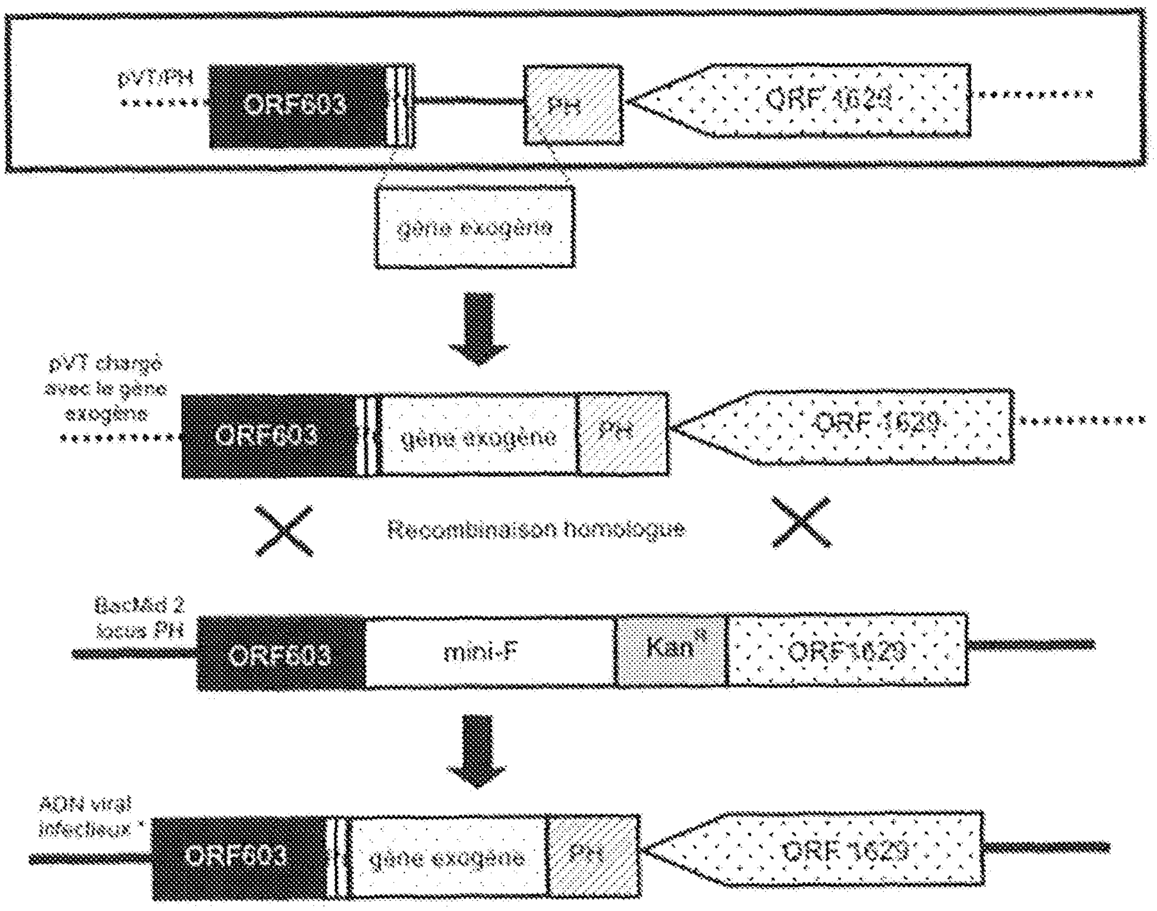
pVT/PH
ORF603
PH
ORF 1629
gène exogène
pVT chargé avec le gène exogène
ORF603
gène exogène
PH
ORF 1629
Recombinaison homologue
BacMid 2 locus PH
ORF603
mini-F
Kan
ORF1629
ADN viral infectieux
ORF603
gène exogène
PH
ORF 1629

Figure 6

1. Construction d'un pVT/gp37 spécifique (pVT/gp37-Cγ1)
Ce vecteur de transfert contient la cassette d'expression suivante :

. Promoteur viral P10

. Séquence d'ADN codant pour une séquence signal d'une immunoglobuline humaine (séquence de sécrétion)

. 2 sites de restriction uniques pour le clonage en phase de la région variable (VH) de l'anticorps (région qui donne la spécificité de l'anticorps)

. Séquence d'ADN qui code une région constante d'IgG (γ1-4,) epsilon, mu, ou alpha humaine.

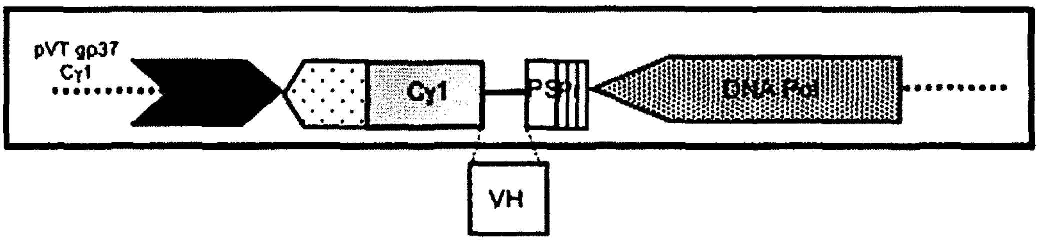


2. Insertion du ADNc codant la région variable de l'anticorps (VH). Il y a alors reconstitution de l'ADNc codant la totalité de la chaine lourde

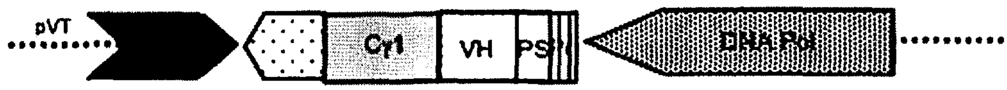


3. Génération des baculovirus recombinants. Recombinaison entre le pVTgp37 chargé avec la chaine lourde + le pVTPH chargé avec la chaine légère + Bacmid 2

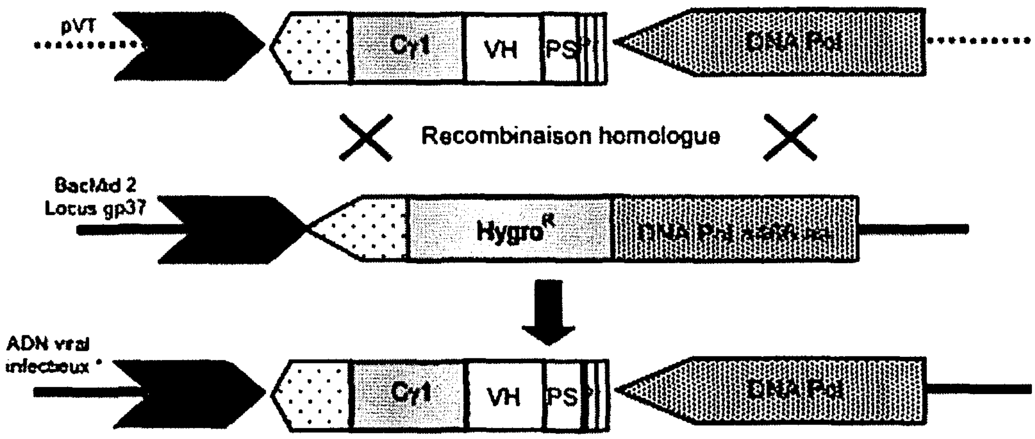


(*) ADN viral infectieux si la recombinaison dans le locus PH s'est produite également.

Figure 7

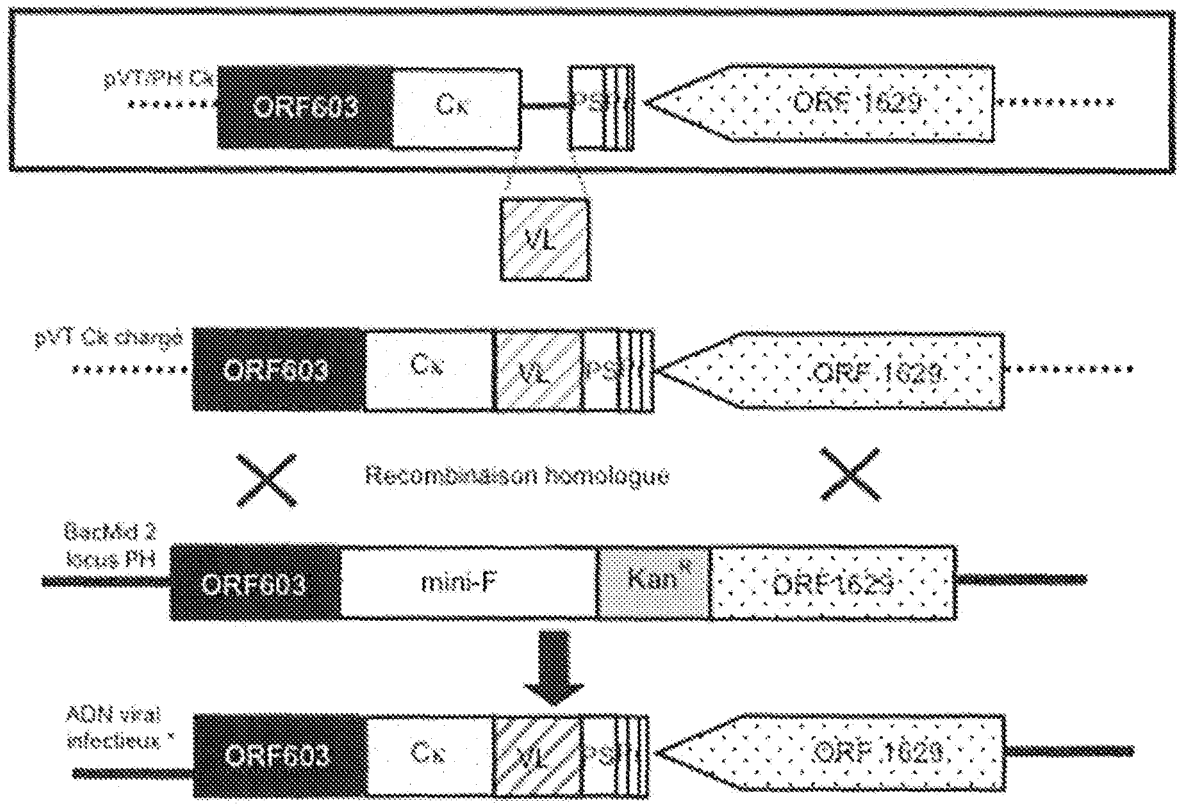


(*) ADN viral infectieux si la 2ème recombinaison locus gp37 s'est produite également.

Figure 8

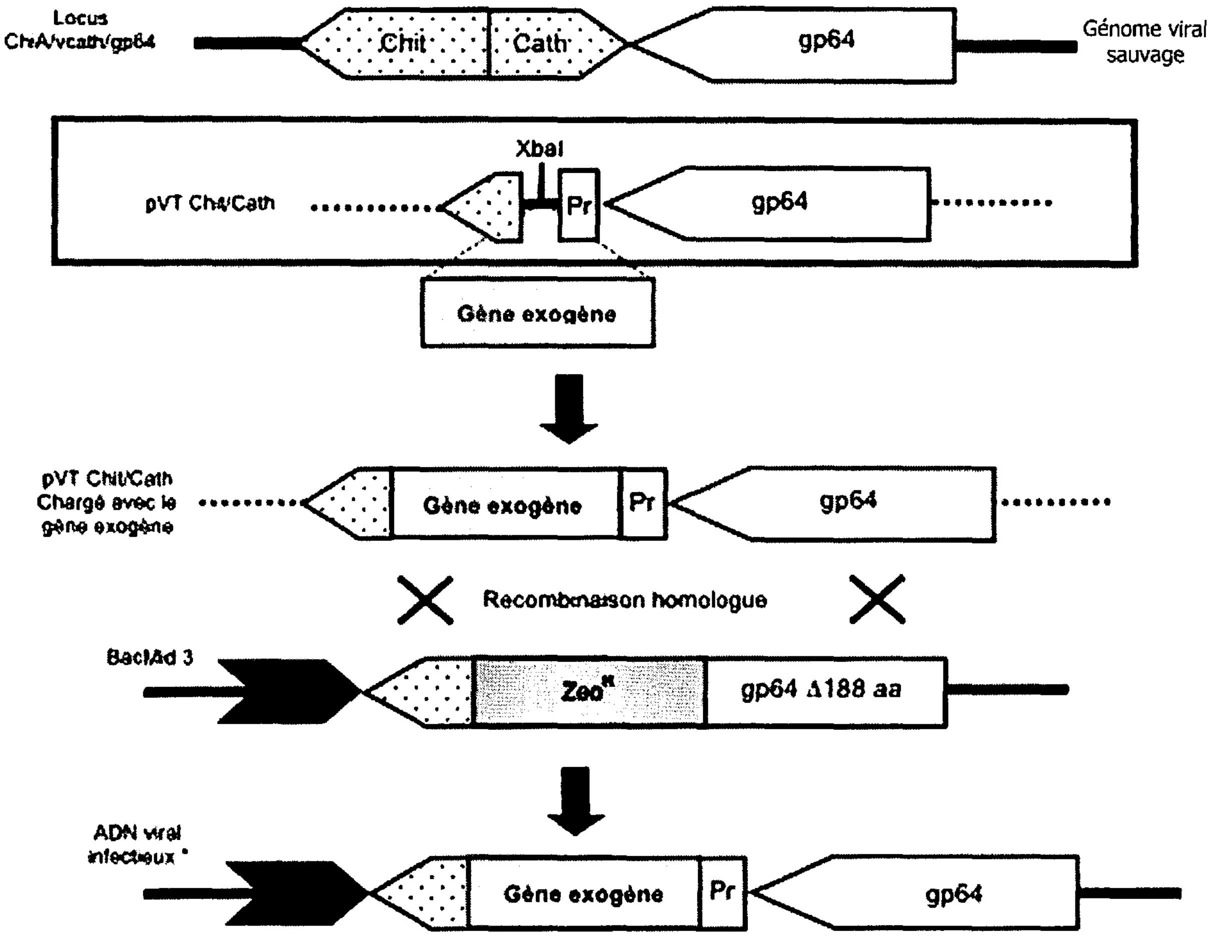


(*) ADN viral infectieux si les 2 autres recombinaisons, effectuées respectivement dans les locus PH et gp37 se sont produites également.

Figure 9

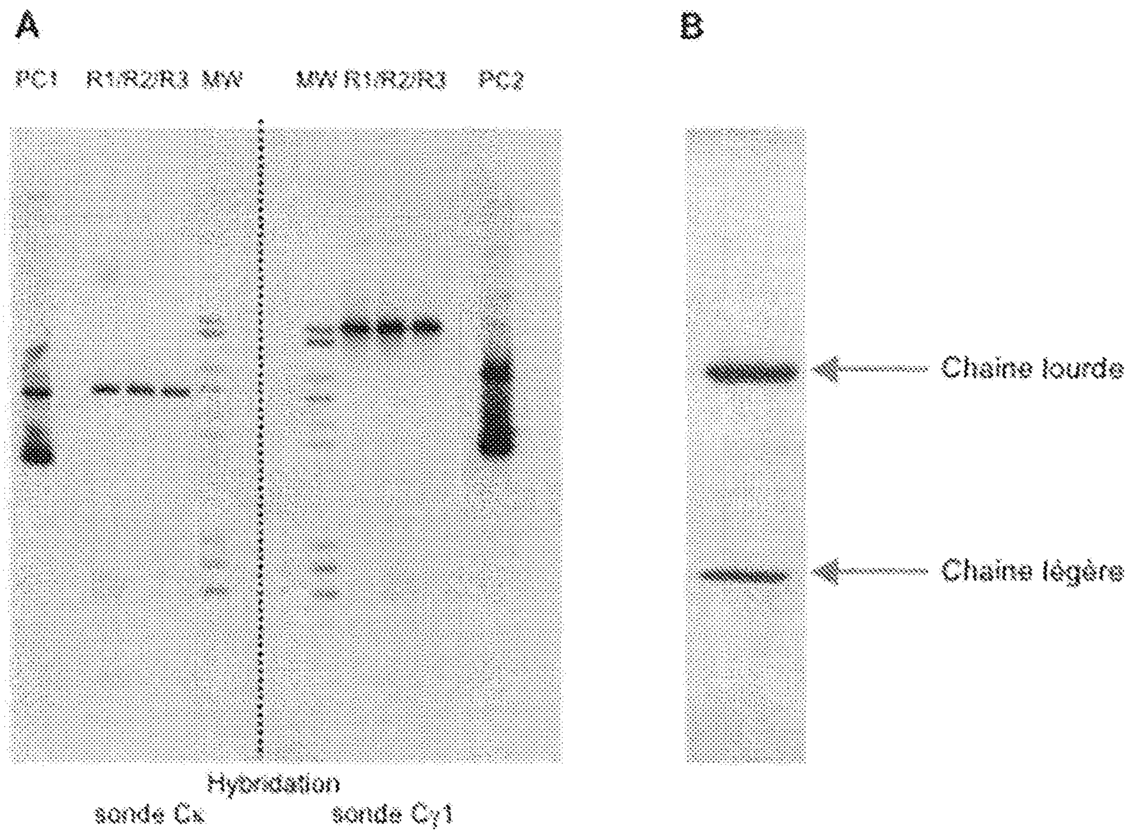
A
PC1 R1/R2/R3 MW MW R1/R2/R3 PC2
Hybridation
sonde Cκ
sonde Cγ1
B
Chaine lourde
Chaine légère

Figure 10

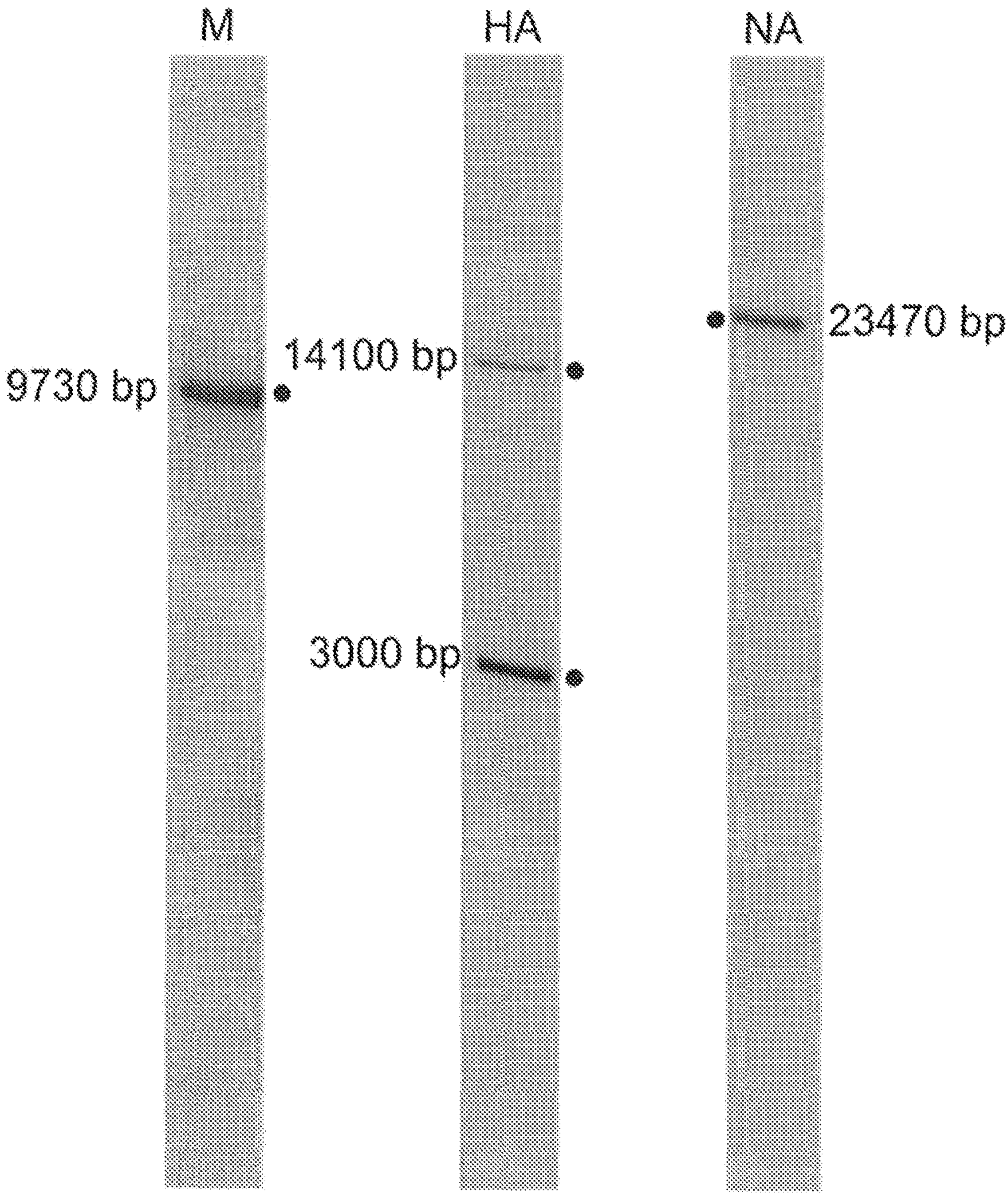

Figure 11

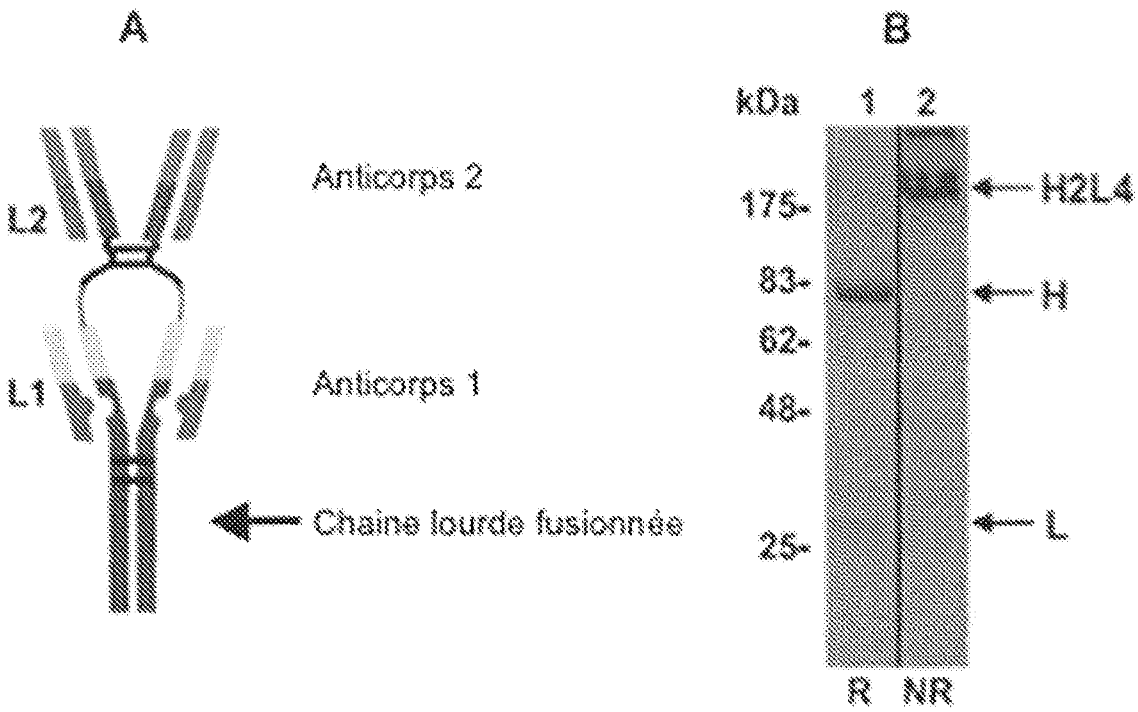
A
L2
Anticorps 2
L1
Anticorps 1
Chaine lourde fusionnée
B
kDa
1
2
175-
83-
62-
48-
25-
H2L4
H
L
R
NR

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 0112829 A, Possee


### Littérature non-brevet citée dans la description


- **GAY N.J. et al.** *Biochem J.,* 1986, vol. 234, 111-117
- **DROCOURT et al.** *Nucleic Acids Research,* 1990, vol. 18 (13
- **GENTZ ; BUJARD.** *J. Bacteriology,* 1985, vol. 164 (1
- **KWON et al.** *J Biol. Chem.,* 1999, vol. 274 (41

SEQUENCE LISTING

<110> CENTRE NATIONAL DE LA RECHERCHE SCIENTIFIQUE (CNRS) et INSTITUT NATIONAL DE LA RECHERCHE AGRONOMIQUE (INRA)

<120> Système d'expression baculovirus

<130> 1H170260 0415

<160> 3

<170> PatentIn version 3.5

<210> 1
<211> 75
<212> DNA
<213> Artificial Sequence

<220>
<223> P10 sauvage

<400> 1

**ataagaatta ttatcaaatc atttgtatat taattaaaat actatactgt aaattacatt 60**

**ttatttacaa tcatg 75**

<210> 2
<211> 71
<212> DNA
<213> Artificial Sequence

<220>
<223> P10S1A

<400> 2

**ataagtattt taatcttttc gtttgtataa attaatttat actatactgt ataaaaaaac 60**

**ctataaatat g 71**

<210> 3
<211> 71
<212> DNA
<213> Artificial Sequence

<220>
<223> P10S1B

<400> 3

```
ataagtattt taatcttttc gtttgtacca ctgcagtggt actatactgt ataaaaaaac        60
ctataaatat g                                                             71
```